(19) 

Europäisches
Patentamt

European
Patent Office

Office européen
des brevets

(11) **EP 4 670 784 A1**

(12) **EUROPEAN PATENT APPLICATION**

(43) Date of publication:
**31.12.2025 Bulletin 2026/01**

(21) Application number: **24185454.6**

(22) Date of filing: **28.06.2024**

(51) International Patent Classification (IPC):
*A61P 7/00* (2006.01)     *A61K 38/00* (2006.01)
*A61K 38/18* (2006.01)     *C07K 14/535* (2006.01)

(52) Cooperative Patent Classification (CPC):
**C07K 14/535; A61K 38/00; A61K 38/18; A61P 7/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Eberhard Karls Universität Tübingen
(Medizinische Fakultät)
72074 Tübingen (DE)**
• **Max-Planck-Gesellschaft zur Förderung der
Wissenschaften e.V.
80539 München (DE)**

(72) Inventors:
• **Skokowa, Julia
72127 Kusterdingen (DE)**
• **ElGamacy, Mohammad
72076 Tübingen (DE)**
• **Ullrich, Timo
72076 Tübingen (DE)**

(74) Representative: **Witte, Weller & Partner
Patentanwälte mbB
Postfach 10 54 62
70047 Stuttgart (DE)**

(54) **G-CSFR-BINDING PROTEIN**

(57)    The present invention relates to a polypeptide configured to assemble into a binder of granulocyte-colony stimulating factor receptor (G-CSFR), a protein comprising said polypeptide, a nucleic acid molecule encoding said polypeptide or protein, an expression vector comprising the nucleic acid molecule, a recombinant host cell comprising said polypeptide, protein, nucleic acid molecule and/or expression vector, a pharmaceutical composition comprising the said polypeptide, protein, nucleic acid molecule, expression vector and/or host cell, and to a kit.

EP 4 670 784 A1

**Description**

The present invention relates to a polypeptide configured to assemble into a binder of granulocyte-colony stimulating factor receptor (G-CSFR), a protein comprising said polypeptide, a nucleic acid molecule encoding said polypeptide or protein, an expression vector comprising the nucleic acid molecule, a recombinant host cell comprising said polypeptide, protein, nucleic acid molecule and/or expression vector, a pharmaceutical composition comprising the said polypeptide, protein, nucleic acid molecule, expression vector and/or host cell, and to a kit.

BACKGROUND OF THE INVENTION

Granulocyte colony-stimulating factor (G-CSF), also known as colony-stimulating factor 3 (CSF 3), is a glycoprotein that stimulates the bone marrow to produce granulocytes and stem cells and release them into the bloodstream. Functionally, it is a cytokine and hormone, a type of colony-stimulating factor, and is produced by a number of different tissues.

The natural receptor of G-CSF, granulocyte-colony stimulating factor receptor (G-CSFR), is activated through ligand-induced dimerization of the ectodomains of two receptor subunits, which leads to the cross-phosphorylation and activation of the intracellular receptor-associated kinases. The native ligand G-CSF engages and dimerizes G-CSFR through two binding sites in a crossover 2:2 receptor:ligand complex that juxtaposes the receptor subunits into a signaling-competent configuration. Physiologically, G-CSFR signaling is primarily responsible for the proliferation and differentiation of hematopoietic stem cells into neutrophils, and for mobilization of hematopoietic cells and neutrophils into peripheral blood from the bone marrow. Additionally, it has pro-proliferative and pro-survival effects on neuronal, vascular, and myocardial tissues. Conversely, G-CSFR signaling promotes several types of leukemia and solid tumors. It also constitutes a driver of inflammatory disorders, including rheumatoid arthritis.

Due to its clinical relevance, binding G-CSFR and thereby inhibiting G-CSFR activity represents a promising therapeutic approach. The currently available binders are limited to anti-G-CSFR antibodies; see, e.g., Scalzo-Inguanti et al. "164: CSL324, a humanised anti G-CSFR antibody, can inhibit neutrophil migration while not impaction on neutrophil number or effector functions." Cytokine 70.1 (2014): 68; ScalzoInguanti et al. "A neutralizing anti-G-CSFR antibody blocks G-CSF-induced neutrophilia without inducing neutropenia in nonhuman primates." Journal of leukocyte biology 102.2 (2017): 537-549; McRae et al. "Blockade of the G-CSF Receptor Is Protective in a Mouse Model of Renal Ischemia-Reperfusion Injury." The Journal of Immunology 205.5 (2020): 1433-1440; Gamell et al. "CSL324, a granulocyte colony-stimulating factor receptor antagonist, blocks neutrophil migration markers that are upregulated in hidradenitis suppurativa." British Journal of Dermatology 188.5 (2023): 636-648; and WO2012171057A1.

However, the use of antibodies as G-CSFR binders has a number of disadvantages. Antibodies have a substantial molecular size of approximately 150 kDa, resulting in high costs due to both their production and the need for cold chain storage. Furthermore, the known antibodies are characterized by low thermostabilities. Importantly, because of the size of the compound, less tumor penetration is expected. All these properties significantly limit the therapeutic applicability of current G-CSFR inhibitors.

It is therefore one of the underlying objectives of the invention to provide new G-CSFR binders with which the problems or disadvantages of the current binders can be reduced and possibly even avoided.

SUMMARY OF THE INVENTION

The object underlying the invention is solved by the provision of a polypeptide configured to assemble into a binder of granulocyte-colony stimulating factor receptor (G-CSFR), which is a non-immunoglobulin-derived polypeptide.

The object underlying the invention is also solved by the provision of a protein comprising the polypeptide according to the invention.

According to the invention, the term "polypeptide" refers to a series of amino acid residues, connected one to the other typically by peptide bonds between the alpha-amino and carbonyl groups of the adjacent amino acids. The length of the polypeptide is not critical to the invention as long as the correct epitopes are maintained, e.g., the epitope(s) which bind(s) to the granulocyte-colony stimulating factor receptor (G-CSFR). The term "polypeptide" is meant to refer to molecules containing more than about 30 amino acid residues. In particular, the polypeptides according to the invention comprise, in certain embodiments, approx. 60 - 180 amino acids, preferably, 80 - 160 amino acids further preferably approx. 90 - approx. 150 amino acids, further preferably of approx. 100 - approx. 140 amino acids, further preferably of approx. 110 - approx. 130 amino acids, most preferably of approx. 124 amino acids. According to the invention, the term "protein" as used herein, describes a macromolecule comprising one or more polypeptide chains.

According to the invention, a "binder" refers to a substance that specifically binds to a target molecule. In particular, it refers to a protein that specifically binds to G-CSFR, preferably, but not exclusively, to the binding site for natural G-CSFR. The binding of the binder to G-CSFR may result in G-CSFR remaining inhibited, activated or unaffected with respect to its activity.

According to the invention, "specifically binding to" is defined as the general interaction between two molecules where one molecule (the binder) recognizes and binds to a distinct and predefined region (epitope) on the other molecule (the target), with a significantly higher affinity and selectivity compared to interactions with other molecules or epitopes. Specifically, the binder is represented by the binder according to the invention, and the target is G-CSFR. The binding occurs under stringent conditions that mimic physiological conditions or under enhanced conditions that further discriminate specific from non-specific interactions. Stringent conditions may include factors such as temperature, ionic strength, and pH that are optimized to reduce or prevent the binding of molecules that interact weakly or non-specifically. A specific binding can be determined via various measurements or assays, e.g. affinity measurements, competition assays, mutational analyses, etc.

According to the invention "assembly" refers to a process by which a polypeptide chain folds and converts to form a functional protein structure. In particular, it refers to a biochemical process where, under suitable conditions, one chain of the polypeptide of the invention folds into a distinct and stable three-dimensional structure that constitutes a functional binder protein. This process may include the transition from linear sequences of amino acids (primary structure) to complex structures involving alpha helices and beta sheets (secondary structures), which further fold into unique three-dimensional configurations (tertiary structures). In multimeric proteins, multiple polypeptides (subunits) associate to form a functional complex (quaternary structure). It may also involve non-covalent interactions such as hydrogen bonds, ionic bonds, hydrophobic interactions, and van der Waals forces primarily drive the assembly process. In some cases, covalent disulfide bonds may also stabilize the structure.

According to the invention, the granulocyte-colony stimulating factor receptor (G-CSFR) also known as CD114 (Cluster of Differentiation 114) is a cell-surface receptor for the granulocyte colony-stimulating factor (G-CSF). The invention includes all isoforms. In particular, it is referred to human G-CSFR.

According to the invention "non-immunoglobulin-derived" means that the polypeptide does not include an antibody, T cell receptor, or fragments thereof. The polypeptide and peptide according to the invention are, therefore, non-antibody polypeptides and/or non-antibody proteins, respectively.

The inventors have recognized that it is possible to provide a peptidic, non-antibody G-CSFR binder. It is characterized by its significantly reduced size, usually of $\leq 50$ kDa, preferably $\leq 40$ kDa, more preferably $\leq 30$ kDa and most preferably $\leq 20$ kDa or even $\leq 15$ kDa. The small size is accompanied by significantly easier and more cost-effective manufacturability and significantly increased thermostability.

In an embodiment of the invention the peptide or protein is monomeric and, in a further embodiment, the peptide or protein lacks posttranslational modifications and can be readily purified, e.g., from *Escherichia coli.* These and aforementioned properties allow for lower production costs, simplified storage conditions, and higher tissue or tumor penetration capabilities when compared to the known anti-G-CSFR antibodies. In addition, the compactness of the binder allows its seamless integration into other protein constructs, e.g., as a homing tag.

For this reason, in one embodiment, a compound is bound to the polypeptide and/or protein according to the invention. Thus, the polypeptide or protein according to the invention serves as a transporter or vehicle to deliver the bound compound to the G-CSFR. In particular, compounds can be delivered "piggybacked" on the polypeptide or protein to G-CSFR-expressing cells, such as tumor cells, in this manner. The coupled compounds can be chemically or biologically defined substances, such as active agents, dyes, polypeptides, proteins, antibodies, etc. In one embodiment of the invention, the bound compound is a signaling molecule such as a cytokine or chemokine.

It is self-evident to a person skilled in the art that all features, properties, advantages, etc., disclosed for the polypeptide according to the invention apply correspondingly to the protein according to the invention, without the need for explicit reference thereto.

The problem underlying the invention is hereby completely solved.

Biophysical measurements of the inventors demonstrated a low nanomolar affinity to G-CSFR allowing the binder to effectively outcompete the natural ligand G-CSF at nanomolar concentrations. Its binding activity was demonstrated in several *in vitro* assays including G-CSF-stimulated acute myeloid leukemia cells and hematopoietic stem and progenitor cells. Because G-CSF is the main cytokine inducing neutrophil production, activation and survival, binding to G-CSFR represents a promising therapeutic approach for the treatment of autoimmune diseases, inflammatory conditions, and infections, that are connected to neutrophilia and/or hyperactivated neutrophils. Moreover, because several types of cancers, such as leukemia, breast cancer, brain tumors, are dependent on autocrine or paracrine G-CSF:G-CSFR signaling, binding of G-CSFR by the new peptide may have potential anti-tumorigenic activity.

In an embodiment of the invention the binder is an inhibitor of G-CSFR.

This measure adapts the polypeptide or protein according to the invention to a clinically particularly relevant application. The activation of G-CSFR and the associated signaling is responsible for the proliferation and differentiation of cells that express the receptor. Tumor cells in particular make use of this signaling. By inhibiting the G-CSFR, the signaling chain is interrupted and the tumor cell no longer receives any stimuli. The polypeptide and protein according to the invention thus becomes a particularly effective and efficient anti-tumor agent.

According to the invention, an "inhibitor" generally refers to a substance that decreases or prevents the activity of a target molecule. Specifically, it refers to a protein that decreases or prevents the activity of G-CSFR by blocking its ability to being bound and activated by its ligand G-CSF. This inhibition can be achieved through the inhibitor specifically binding directly to a specific part of G-CSFR that normally interacts with its ligand. By doing so, the inhibitor effectively blocks the binding site, preventing the G-CSF from attaching to and activating its receptor G-CSFR.

In an embodiment of the invention said polypeptide comprises an amino acid chain having approx. 60 - approx. 180 amino acids, preferably approx. 80 - approx. 160 amino acids, further preferably approx. 90 - approx. 150 amino acids, further preferably of approx. 100 - approx. 140 amino acids, further preferably of approx. 110 - approx. 130 amino acids, most preferably of approx. 124 amino acids.

Of particular advantage is that the polypeptides according to the invention are relatively short and small, which facilitates their production and assembly into a protein.

In yet another embodiment of the invention the polypeptide comprises the following amino acid sequence SEQ ID NO: 1:

MAAL*DAA*LYEIYDGL*IL*YQQRLKSLEGISPELGPALDALRX$_1$X$_2$MAX$_3$FAX$_4$X$_5$

MAQAMEEGLDSLPQX$_6$FLX$_7$X$_8$ALX$_9$X$_{10}$IRX$_{11}$IQADAAALREKLAATYKGNDR

AAA*AQSIARK*LE*EM*LEKAYQILRHLAAA,

wherein

$X_1 = Y, L, W$
$X_2 = D$
$X_3 = D$
$X_4 = I, R, Y, C$
$X_5 = L$
$X_6 = R, 5$
$X_7 = R, Y, W$
$X_8 = K$
$X_9 = E$
$X_{10} = M$
$X_{11} = K, W.$

The inventors were able to identify a consensus amino acid sequence that yields polypeptides that have the desired properties. This measure has the advantage that a person skilled in the art has a range of variation options. That is, at positions $X_1$-$X_{11}$ the respective amino acids indicated can be used as desired. Such amino acid residues are, according to the findings of the inventors, in an embodiment, responsible for enhancing the receptor-binding affinity, and thus the binding or inhibitory potency. It is understood that according to the invention, all arbitrary and conceivable combinations of $X_1$-$X_{11}$ as indicated are encompassed and disclosed.

The amino acid residues shown in cursory form are, according to the findings of the inventors, in an embodiment responsible for the monomeric nature of the assembled binder.

In another embodiment the polypeptide according to the invention comprises the following amino acid sequence SEQ ID NO: 2:

MAALDAALYEIYDGLILYQQRLKSLEGISPELGPALDALRYDMADFAYLMAQAMEEGLDS

LPQRFLWKALEMIRKIQADAAALREKLAATYKGNDRAAAAQSIARKLEEMLEKAYQILRHL

AAA ("bop3")

or the following amino acid sequence SEQ ID NO: 3:

MAALDAALYEIYDGLILYQQRLKSLEGISPELGPALDALRYDMADFAILMAQAMEEGLDSL

PQSFLRKALEMIRKIQADAAALREKLAATYKGNDRAAAAQSIARKLEEMLEKAYQILRHLA

AA ("bop1")

or the following amino acid sequence SEQ ID NO: 4:

MAALDAALYDIYDGLILYQQQLESLEGISPELGPALDALRYDMADFAILMAQAMEEGLDSL PQSFLRKALEMIRKIQADAAALREKLAATYKGNDRAAAAQSIARKLEEMLRKAYQDLRHL AAA ("bop2")

or the following amino acid sequence SEQ ID NO: 5:

MAALAAALQSIYRKLAEMQAKLKSLEGISPELGPALDALRYDMADFAILMAQAMEEGLDS LPQSFLRKALEMIRKIQADAAALREKLAATYKGNDRADAAVYIAADLEILLEQAYQILRHLA AA ("boa1")

or the following amino acid sequence SEQ ID NO: 6:

MAALAAALQSIYRKLAEMQRKLKSLEGISPELGPALDALRYDMADFAILMAQAMEEGLDS LPQSFLRKALEMIRKIQADAAALREKLAATYKGNDRADAAVYDAADLEILLEQAYEILRHLA AA ("boa2")

or the following amino acid sequence SEQ ID NO: 7:

MAALAAALAEIYKGLAEYQARLKSLEGISPELGPALDALRWDMADFAYLMAQAMEEGLD SLPQRFLWKALEMIRKIQADAAALREKLAATYKGNDRAAAAVEIAAQLEAFLEKAYQILRH LAAA ("bv21")

or the following amino acid sequence SEQ ID NO: 8:

MAALAAALAEIYKGLAEYQARLKSLEGISPELGPALDALRYDMADFAYLMAQAMEEGLDS LPQRFLWKALEMIRKIQADAAALREKLAATYKGNDRAAAAVEIAAQLEAFLEKAYQILRHL AAA ("bv22")

or the following amino acid sequence SEQ ID NO: 9:

MAALAAALAEIYKGLAEYQARLKSLEGISPELGPALDALRYDMADFAYLMAQAMEEGLDS LPQSFLWKALEMIRWIQADAAALREKLAATYKGNDRAAAAVEIAAQLEAFLEKAYQILRHL AAA ("bv23")

or the following amino acid sequence SEQ ID NO: 10:

MAALAAALAEIYKGLAEYQARLKSLEGISPELGPALDALRWDMADFACLMAQAMEEGLD SLPQRFLWKALEMIRKIQADAAALREKLAATYKGNDRAAAAVEIAAQLEAFLEKAYQILRH LAAA ("bv24").

This measure provides specific G-CSFR binders or polypeptides, designated by the inventors as 'bop3', 'bop1', 'bop2', 'boa1', 'boa2', 'bv21', 'bv22', 'bv23', and 'bv24", which are particularly suitable according to their findings in accordance with the invention. According to the inventors' findings, the binders 'bop1' and 'bop3' exhibit particularly high inhibitory activity towards G-CSFR. 'boa1' and 'boa2' also exhibit G-CSFR inhibitory activity. 'bop2' is a strong activator of G-CSFR. 'bv21', 'bv22', 'bv23' and 'bv24' are characterized by a high affinity for G-CSFR.

In a particularly preferred embodiment of the invention the polypeptide consists or consists essentially of an amino acid sequence according to SEQ ID NO: 1 to SEQ ID NO: 10.

"Consisting essentially of" shall mean that a polypeptide according to the present invention, in addition to the sequence according to any of SEQ ID NO: 1 to SEQ ID NO: 10 contains additional N- and/or C-terminally located stretches of amino acids that are not necessarily forming part of the polypeptide that functions as a G-CSFR binding epitope.

The polypeptides and/or proteins and/or nucleic acid molecules disclosed in accordance with the present invention may also be in "purified" form. The term "purified" does not require absolute purity; rather, it is intended as a relative definition, and can include preparations that are highly purified or preparations that are only partially purified, as those terms are understood by those of skill in the relevant art.

The polypeptides, proteins and nucleic acids according to the invention may be in "enriched form". As used herein, the term "enriched" means that the concentration of the material is at least about 2, 5, 10, 100, or 1000 times its natural concentration (for example), advantageously 0.01 %, by weight, preferably at least about 0.1% by weight. Enriched preparations of about 0.5%, 1%, 5%, 10%, and 20% by weight are also contemplated. The sequences, constructs, vectors, clones, and other materials comprising the present invention can advantageously be in enriched or isolated form.

In an embodiment of the invention the protein comprises one polypeptide having any of the following amino acid sequences: SEQ ID NO: 1, SEQ ID NO: 2 (bop3), SEQ ID NO: 3 (bop1), SEQ ID NO: 4 (bop2), SEQ ID NO: 5 (boa1), SEQ ID NO: 6 (boa2), SEQ ID NO: 7 (bv21), SEQ ID NO: 8 (bv22), SEQ ID NO: 9 (bv23), SEQ ID NO: 10 (bv24).

This embodiment takes into account the inventors' realization that the proteins or polypeptides according to the invention are preferably monomeric in solution.

Another subject-matter of the invention relates to the polypeptide and/or the protein according to the invention for use as a medicament, preferably in the treatment of a disease, further preferably selected from the group consisting of: cancer, including leukemia and acute myeloid leukemia; cardiovascular disease; inflammatory disease, including rheumatoid arthritis; and neurologic disease.

In another embodiment the protein according to the invention comprises: (a) one polypeptide chain, (b) a bundle of four $\alpha$-helices; and (c) three amino acid linkers that connect contiguous bundle-forming $\alpha$-helices that are located on the polypeptide chain, wherein each amino acid linker has a length between 2 and 15 amino acids; wherein the protein comprises one or more G-CSFR binding sites, and wherein the protein has a melting temperature ($T_m$) of at least 90 °C.

The term "$\alpha$-helix" as used herein, indicates a right-handed spiral conformation of a polypeptide chain or of a part of a polypeptide chain. In an $\alpha$-helix, every backbone N-H group donates a hydrogen bond to the backbone C=0 group of the amino acid three or four or five residues earlier along the polypeptide chain.

A "bundle of four $\alpha$-helices" as used herein, is defined as a protein fold composed of four $\alpha$-helices that are nearly parallel or antiparallel to each other. An $\alpha$-helix that contributes to the bundle of four $\alpha$-helices is called a "bundle-forming $\alpha$-helix". The four $\alpha$-helices that form the bundle of four $\alpha$-helices are located on a single polypeptide chain.

In the protein according to the invention an amino acid linker connects two $\alpha$-helices that are located on the same polypeptide chain. The term "amino acid linker" as used herein, refers to a sequence of amino acids that is located between the C-terminal end of a first $\alpha$-helix and the N-terminal end of a second $\alpha$-helix, wherein the amino acids of the amino acid linkers are not part of any of the $\alpha$-helices. Two $\alpha$-helices are said to be contiguous because they are located on the same polypeptide chain and are directly connected by an amino acid linker. The length of an amino acid linker is defined as the number of amino acid residues that constitute the linker.

The term "binding site", as used herein, refers to one or more regions of the protein according to the invention that, as a result of its shape, favorably associate with another chemical entity or compound. A "G-CSFR binding site" as used herein, refers to one or more regions of the protein that favorably associate with G-CSFR. The shape of a protein-based binding site is determined by a set of amino acids with specific molecular interaction features and a defined spatial arrangement towards each other.

The skilled person is aware of methods to determine structural features of a protein such as $\alpha$-helices or beta-sheets and/or linker sequences between such structures. The most common methods to determine the three-dimensional structure of a protein are X-ray crystallography, NMR spectroscopy and cryo-electron microscopy. These methods may be applied to detect the position and lengths of $\alpha$-helices in a protein and the amino acids involved in the formation of these $\alpha$-helices. Further, the methods may be applied to determine the length of amino acid linkers between two contiguous $\alpha$-helices located on the same polypeptide chain and to identify the amino acids that form these linkers (i.e., the position and length of such linkers in the amino acid sequence), if these linkers are structured. In addition, these methods may be applied to determine the orientation of $\alpha$-helices towards each other, for example parallel or antiparallel orientation, within a protein. Further biophysical methods that may be applied to determine secondary structures of proteins include circular dichroism (CD) spectroscopy and Fourier-transform infrared (FTIR) spectroscopy.

Alternatively, structural features of proteins such as, for example, the lengths of $\alpha$-helices and/or amino acid linkers, may be predicted by using computational methods that start from the primary amino acid sequence of a protein. Several computer programs are known in the art that may be applied for the prediction of secondary protein structures. By way of non-limiting example, suitable computer programs include Psipred [McGuffin et al. (2000), The PSIPRED protein structure prediction server, Bioinformatics Vol. 16, Issue 4, pp. 404-405], SPIDER2 [Yang et al. (2016), SPIDER2: A package to predict secondary structure, accessible surface area, and main-chain torsional angles by deep neural networks], PSSPred [https://zhanglab.ccmb.med.umich.edu/PSSpred/], DeepCNF [Wang et al. (2016), Protein secondary structure prediction using deep convolutional neural fields, Scientific Reports 6, 18962]. One or more computer programs may be used for the prediction of a protein structure. Adaptation of the settings may be required to be able to directly compare the results of the different programs. The computer programs may be used in combination with experimental data to refine the results of the computational prediction.

The protein according to the invention has a melting temperature ($T_m$) of at least z90 °C, in an embodiment of at least ≥100 °C, preferably at least ≥110 °C.

This measure has the advantage of providing the protein of the invention in a form that gives sufficient stability to allow it to be processed, formulated and stored for extended periods of time. The protein melting point ($T_m$) is defined as the temperature at which the protein denatures. The temperature at which a protein is fully denatured depends on various factors, for example, the solvent and buffer conditions, a bound ligand, pressure and the temperature ramp rate that is applied to the protein. Within the present invention, the thermal stability of the protein of the invention can be tested in a buffer comprising HEPE and NaCl, pH 7.4 and the temperature was increased at a rate of 1 °C (Celsius) per minute. The melting temperature ($T_m$) may be extracted from a melting curve and corresponds to the temperature at which 50% of the protein is unfolded. Accordingly, the melting temperature is defined as the melting curve inflection mid-point.

In yet another embodiment the protein binds to G-CSFR with a dissociation constant $K_D$ of less than 100 nM, preferably less than 50 nM, further preferably less than 10 nM, further preferably less than 5 nM, further preferably less than 4 nM, further preferably less than 3 nM, further preferably less than 2 nM, further preferably less than 1 nM.

This measure provides a protein with a particularly high affinity for G-CSFR. Binding affinity may be quantified by measuring an (equilibrium) dissociation constant ($K_D$), which refers to the dissociation rate constant ($k_D$, time$^{-1}$) divided by the association rate constant ($k_a$, time$^{-1}$ M$^{-1}$). $K_D$ can be determined by measurement of the kinetics of complex formation and dissociation, e.g., using Surface Plasmon Resonance (SPR) methods, e.g., a Biacore™ system; kinetic exclusion assays such as KinExA®; and BioLayer interferometry (e.g., using the ForteBio® Octet® platform). As used herein, "binding affinity" includes not only formal binding affinities, such as those reflecting 1:1 interactions between a polypeptide and its target, but also apparent affinities for which KJs are calculated that may reflect avid binding.

In an embodiment of the protein according to the invention each amino acid linker has a length of between 2 and 15, preferably between 2 and 10, and most preferably between 3 and 7 amino acids.

The inventors were able to find out that such lengths of the linkers result in optimum binding activity. Without being bound to theory, the shorter linkers may presumably contribute to the improved stability of these protein.

In a further embodiment of the invention the protein has an inhibitory activity of G-CSF-induced proliferation, preferably at a half maximal inhibitory concentration ($IC_{50}$) of approx. 1 - 40 nM, preferably 1 - 30 nM, further preferably 1 - 20 nM.

This measure has the advantage of providing highly potent inhibitors that already exhibit significant activity in the nanomolar range. Half maximal inhibitory concentration ($IC_{50}$) is a measure of the potency of a substance in inhibiting a specific biological or biochemical function. $IC_{50}$ is a quantitative measure that indicates how much of a particular inhibitory substance (e.g. drug) is needed to inhibit, *in vitro,* a given biological process or biological component by 50%. $IC_{50}$ can be determined with functional assays or with competition binding assays, well know to the skilled person.

In yet another embodiment the protein according to the invention has a molecular weight of ≤ 14 kDa.

This measure brings the particular advantages of a low molecular mass to bear, namely simple and inexpensive production, increased stability and thus particular suitability as a drug. According to the invention "≤ 14 kDa" means 14 kDa, 13 kDa, 12 kDa, 11 kDa, 10 kDa etc. and all intermediate values.

In another embodiment of the polypeptide and/or the protein according to the invention the polypeptide chain comprises an amino acid sequence having at least 60%, 70%, 80%, 90% or 100% amino acid sequence identity with an amino acid sequence selected from the group consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 and SEQ ID NO: 10.

In the present invention, the term "identical" or "homologous" refers to the degree of identity between sequences of two amino acid sequences, i.e., peptide or polypeptide sequences. The aforementioned "identity" or, synonymously, "homology" is determined by comparing two sequences aligned under optimal conditions over the sequences to be compared. Such a sequence identity can be calculated by creating an alignment using, for example, the ClustalW algorithm. Commonly available sequence analysis software, more specifically, Vector NTI, GENETYX or other tools are provided by public databases.

"Percent identity", "percent identical", or synonymously "percent homology" or "percent homologous" in turn, when referring to a sequence, means that a sequence is compared to a claimed or described sequence after alignment of the sequence to be compared (the "Compared Sequence") with the described or claimed sequence (the "Reference Sequence"). The percent homology (synonym: percent identity) is then determined according to the following formula:

$$\text{percent identity} = 100 \left[1 - (C/R)\right]$$

wherein C is the number of differences between the Reference Sequence and the Compared Sequence over the length of alignment between the Reference Sequence and the Compared Sequence, wherein

(i) each base or amino acid in the Reference Sequence that does not have a corresponding aligned base or amino acid in the Compared Sequence and

(ii) each gap in the Reference Sequence and

(iii) each aligned base or amino acid in the Reference Sequence that is different from an aligned base or amino acid in the Compared Sequence, constitutes a difference and

(iv) the alignment has to start at position 1 of the aligned sequences;

and R is the number of bases or amino acids in the Reference Sequence over the length of the alignment with the Compared Sequence with any gap created in the Reference Sequence also being counted as a base or amino acid.

If an alignment exists between the Compared Sequence and the Reference Sequence for which the percent identity as calculated above is about equal to or greater than a specified minimum Percent Identity then the Compared Sequence has the specified minimum percent identity to the Reference Sequence even though alignments may exist in which the herein above calculated percent identity is less than the specified percent identity.

Methods for comparing the identity/homology of two or more sequences are known in the art. For example, the "needle" program, which uses the Needleman-Wunsch global alignment algorithm [Needleman and Wunsch (1970), J. Mol. Biol. 48:443-453] to find the optimum alignment (including gaps) of two sequences when considering their entire length may be used. The needle program is for example available on 30 the World Wide Web site and is further described in the following publication [EMBOSS: The European Molecular Biology Open Software Suite (2000) Rice, P. Longden, I. and Bleasby, A. Trends in Genetics 16, (6) pp. 276-277]. The percentage of identity between two polypeptides, in accordance with the disclosure, is calculated using the EMBOSS: needle (global) program with a "Gap Open" parameter equal to 10.0, a "Gap Extend" parameter equal 35 to 0.5, and a Blosum62 matrix.

An amino acid sequence which is "approx. at least 60% and at most approx. 100% identical" refers to an amino acid sequence having, over its entire length, at least about 60%, or more, in particular about 61%, about 62%, about 63%, about 64%, about 65%, about 66%, about 67%, about 68%, about 69%, about 70%, about 71%, about 72%, about 73%, about 74%, about 75%, about 76%, about 775, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, and at most about 98% sequence identity with the entire length of a reference sequence, such as any of the amino acid sequences SEQ ID NOS: 1-10.

Another subject-matter of the invention relates to a nucleic acid molecule encoding for the polypeptide and/or protein according to the invention, optionally linked to a promoter sequence.

As used herein the term "nucleic acid molecule" or, synonymously "polynucleotide" coding for (or encoding) a polypeptide/protein refers to a nucleotide sequence coding for the protein/polypeptide including artificial (man-made) start and stop codons compatible for the biological system the sequence is to be expressed. The term "promoter" means a region of DNA involved in the binding of the RNA polymerase to initiate transcription.

The polynucleotide or nucleic acid molecule coding for said polypeptide or protein may be synthetically constructed or may be naturally occurring. The nucleic acid or polynucleotide may be, for example, DNA, cDNA, PNA, RNA or combinations thereof, either single- and/or double- stranded, or native or stabilized forms of polynucleotides, such as, for example, polynucleotides with a phosphorothioate backbone, and it may or may not contain introns so long as it codes for the polypeptide. Of course, only peptides that contain naturally occurring amino acid residues joined by naturally occurring peptide bonds are encodable by a polynucleotide.

A still further aspect of the invention provides a vector, such as an expression vector, capable of expressing the polypeptide and/or protein according to the invention.

The features, characteristics, advantages, and embodiments disclosed for the polypeptide and protein according to the invention apply to the nucleic acid molecule and (expression) vector correspondingly.

Another subject-matter of the invention relates to a recombinant host cell comprising the polypeptide or protein according to the invention, or the nucleic acid or the expression vector according to the invention, wherein said host cell preferably is selected from a bacterial (e.g., *E. coli*), yeast, insect, mammalian, or human cell.

The features, characteristics, advantages and embodiments disclosed for the polypeptide and protein according to the invention apply to the host cell correspondingly.

Another subject-matter according to the invention relates to a pharmaceutical composition comprising at least one active ingredient selected from the group consisting of the polypeptide according to the invention, the protein according to the invention, the nucleic acid or the expression vector according to the invention, and the recombinant host cell according to the invention, optionally a pharmaceutically acceptable carrier, and further optionally, pharmaceutically acceptable excipients and/or stabilizers. The pharmaceutical composition is preferably designated for the treatment of a disease, further preferably selected from the group consisting of: cancer, including leukemia and acute myeloid leukemia; cardiovascular disease; inflammatory disease, including rheumatoid arthritis; and neurologic disease.

A still further subject-matter according to the invention relates to a kit comprising:

(a) a container comprising the polypeptide, or the protein, or the nucleic acid molecule, or the expression vector, or the recombinant host cell, or the pharmaceutical composition according to the invention, in solution or in lyophilized formulation;

(b) optionally, a second container containing a diluent or reconstituting solution for the lyophilized formulation;

(c) optionally, instructions for (i) use of the solution or (ii) reconstitution and/or use of the lyophilized formulation.

The kit may further comprise one or more of (iii) a buffer, (iv) a diluent, (v) a filter, (vi) a needle, or (v) a syringe. The container is preferably a bottle, a vial, a syringe or test tube; and it may be a multi-use container. The pharmaceutical composition is preferably lyophilized.

The kit of the present invention preferably comprises a lyophilized formulation of the present invention in a suitable container and instructions for its reconstitution and/or use. Suitable containers include, for example, bottles, vials (e.g., dual chamber vials), syringes (such as dual chamber syringes) and test tubes. The container may be formed from a variety of materials such as glass or plastic. Preferably the kit and/or container contain/s instructions on or associated with the container that indicates directions for reconstitution and/or use. For example, the label may indicate that the lyophilized formulation is to be reconstituted to peptide concentrations as described above. The label may further indicate that the formulation is useful or intended for subcutaneous administration.

A still further subject-matter of the invention is the use of the polypeptide according to the invention for reconstituting a G-CSFR-binding and/or G-CSFR-inhibiting protein, preferably a monomeric protein.

The features, characteristics, advantages and embodiments disclosed for the polypeptide or protein recited at the outset according to the invention apply to said use correspondingly.

An integral part of the disclosure is a sequence listing. In the event of discrepancies between the sequence listing and the present description, the present description shall prevail.

The invention is now further described and explained in further detail by referring to the following non-limiting examples and figures:

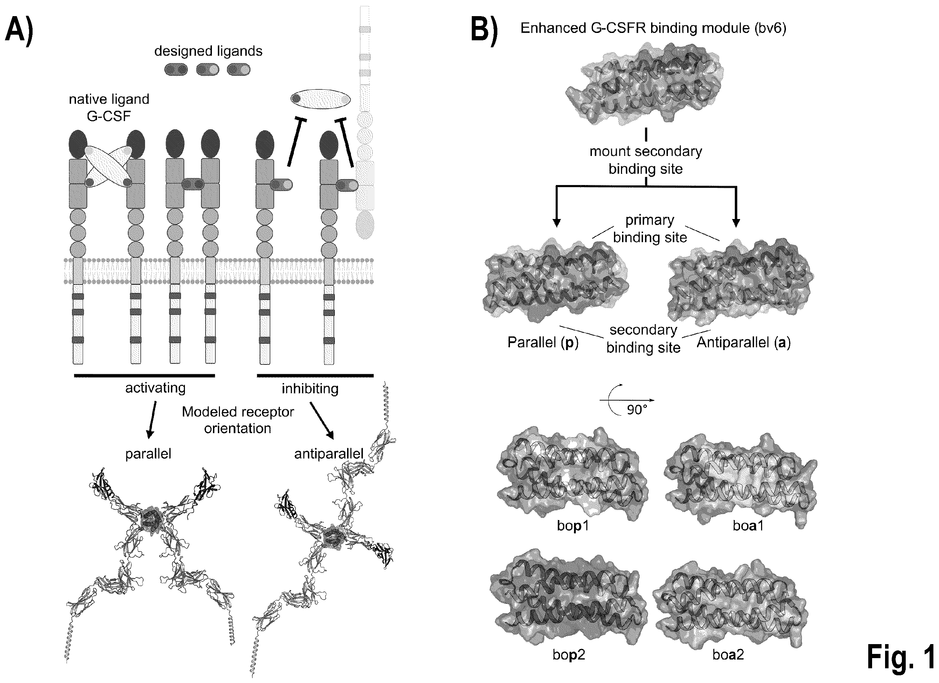

Fig. 1: Design strategy of bifaceted de novo ligands of G-CSFR. (A) G-CSF associates its receptor through two binding sites, illustrated as purple and green dots, that bind the receptor's CRH and Ig-like domains, respectively, resulting in a 2:2 complex. The bifaceted ligands on the other hand encode two (primary and secondary) CRH-binding sites, where the relative orientation and affinity of the secondary binding site defines the biological outcome that can range between activation to inhibition. Under this scheme, the primary binding site (purple dot) has a strong affinity to the receptor and serves as the anchoring point of the ligand. A secondary binding site with sufficient affinity to dimerize the receptor in a parallel orientation (blue dot) in a 2:1 assembly with proximal intracellular segments is conducive of intracellular signaling. Alternatively, signaling inhibitors can be achieved through an antiparallel orientation of the secondary binding site (orange dot) that can result in a strained, unlikely receptor assembly that does not support signaling or stable receptor dimerization. Additionally, inhibitors can also be obtained in cases where the secondary binding site has insufficient affinity to dimerize the receptors, resulting in a 1:1 receptor:ligand complex. (B) Side and front views of the secondary binding site grafts with parallel (bop1 and bop2) or anti-parallel (boa1 and boa2) orientation to the primary binding site. These grafts comprised either a small (bop1 and boa1) or a large (bop2 and boa2) number of residues.

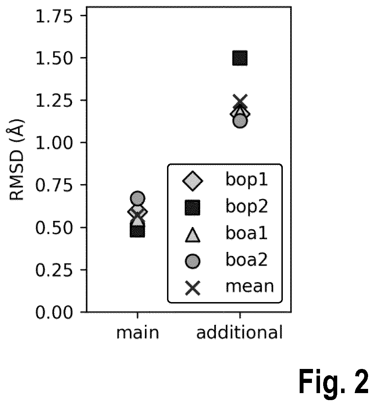

Fig. 2: The G-CSFR-binding site in all design models aligned more poorly to the secondary binding site compared to the primary binding site. AlphaFold2 models of the designed G-CSFR binders bop1, bop2, boa1 and boa2. The corresponding residues of the primary binding site and secondary binding site were structurally aligned to the corresponding residues of G-CSF (from PBD:2D9Q) to determine their backbone atoms RMSD.

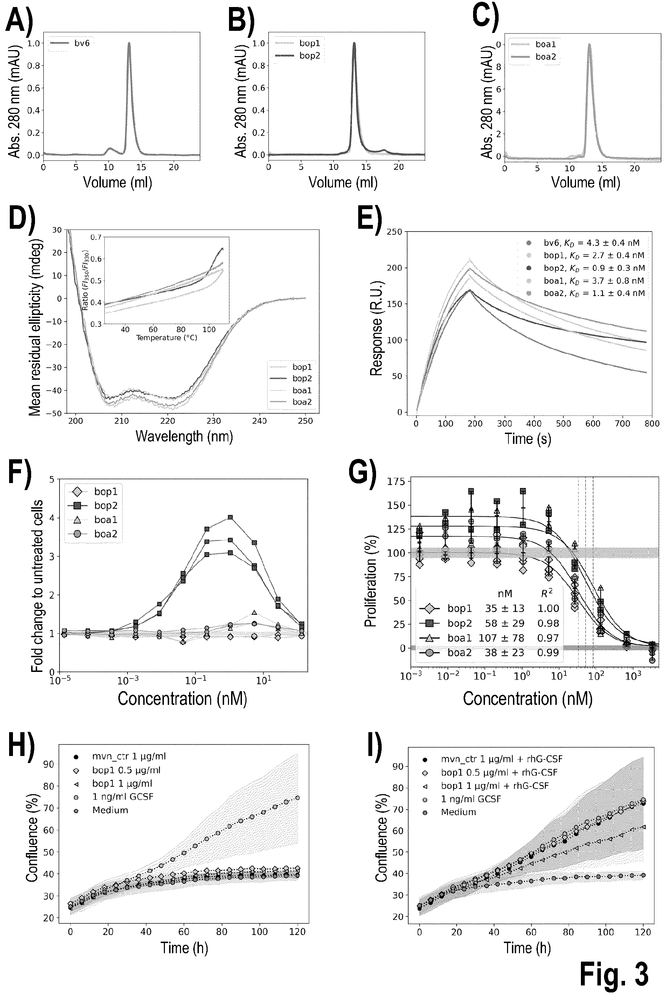

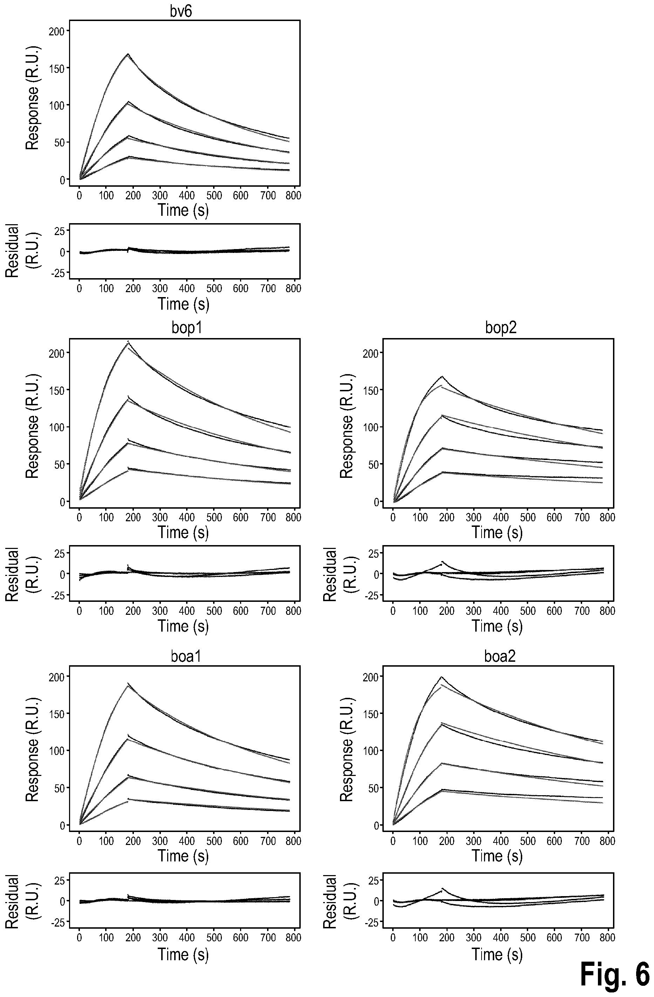

Fig. 3: Characterization of the first generation of bifaceted designs leads to identifying bop1 as an inhibitor with no residual activity. (A, B) Size exclusion chromatography shows the bifaceted designs to be exclusively monomeric proteins. (C) In contrast, the design template bv6 (an enhanced-affinity Boskar4 variant) shows to be minorly dimeric. (D) All the designs have helical CD spectra, and are highly thermostable (inset; nanoDSF melting curves), where the bop designs are less stable (i.e. melting onset at 100 °C) in comparison to the boa designs. NanoDSF plots show the mean of ratio of the fluorescence intensity at 350 nm and 330 nm from 4 technical replicates. (E) SPR sensograms showed that all the designs with the added secondary binding site bound rhG-CSFR with higher affinity in comparison to the starting template, bv6. Particularly, designs with larger binding inter- faces (bop2 and boa2) bound 4-fold tighter than bv6. The binding affinity (KD) values were obtained from fitting 2-fold dilution series performed under the same conditions (Fig. 6, Table 2). (F) The proliferative activity (fold-change to untreated cells) of the designs in NFS-60 cells indicated bop2 to be the most active, whereas boa1 and boa2 had low residual activity, and bop1 had no detectable residual activity across the entire concentration range. The proliferation results were obtained from at least two independent biological replicates. (G) The bop1 design was the most potent inhibiting G-CSF-induced proliferation in competitive inhibition assays of NFS-60 cells. Unlike other designs, bop1 had no residual activity at sub-inhibitory concentrations. A constant concentration of rhG-CSF (50 pg/mL; 100% proliferation) was used in all conditions, where the green and gray shades cover the mean and standard deviation of normalized cell proliferation with and without rhG-CSF, respectively. At least two biological replicas were performed for each design, where half-maximal inhibitory concentrations ($IC_{50}$) values were obtained from the corresponding fit. (H, I) HSCP proliferation assays using 0.5 $\mu$g/ml and 1 $\mu$g/mL of bop1 where performed on healthy donors' CD34[+] HSPCs either without (H) or with the addition of 1 ng/mL rhG-CSF (I). As a negative control, 1 $\mu$g/mL of Moevan_control (mvn_ctrl) was tested, which is a protein lacking a functional G-CSFR binding site. Shown is mean and standard deviation of two independent replicates of three technical replicates each.

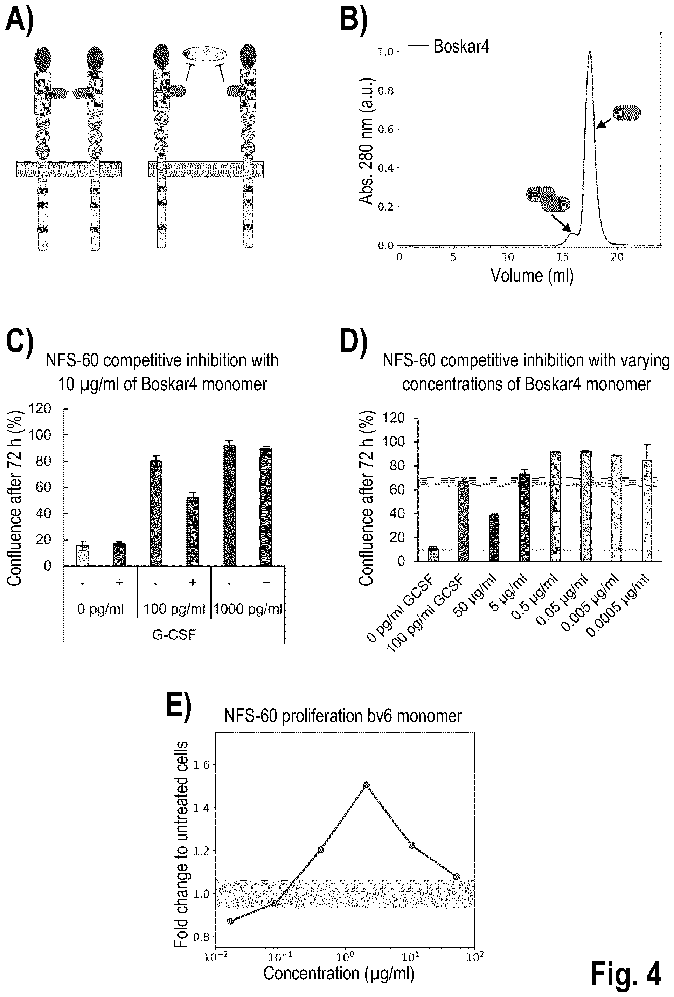

Fig. 4: Oligomeric state of Boskar4 in solution dictates its effect on either activating or inhibiting G-CSFR. (A) The illustration demonstrates how Boskar4 in a two-copy tandem connected by a short linker (Boskar4_st2) can dimerize the receptor subunits (left), while the monomeric fraction can inhibit G-CSF-induced receptor dimerization (right). (B) Analytical SEC shows the Boskar4 design to partition between a dimer and monomer in solution. Chromatography was performed using a Superdex® 200 Increase 10/300 GL analytical column. (C) The monomeric fraction of Boskar4 could to some extent inhibit G-CSF-induced proliferation of NFS-60 cells. The figure shows the results of testing 10 $\mu$g/mL Boskar4 in a cell-based (NFS-60) competitive inhibition assay against 0 pg/mL, 100 pg/mL and 1000 pg/mL of G-CSF, where Boskar4 could outcompete 100 pg/mL G-CSF as indicated by an observed proliferation reduction. (D) Varying concentrations of Boskar4 titrated against 100 pg/mL G-CSF in the same cell assay explained also showed a clear reduction in proliferation at high concentration of monomeric Boskar4 (50 pg/mL). However below non-inhibiting concentrations, an increase in proliferation was clear. Confluence values shown in (C, D) where obtained using the IncuCyte S3 Live-Cell Analysis System, where the bar plots show mean and standard deviation of three technical replicates. (E) Proliferative activity for non-inhibiting concentrations were also observed in NFS-60 proliferation assays for the affinity enhanced Boskar4 variant bv6 (dark gray circles). Shown is the fold change to untreated cells. The proliferation level of untreated cells is indicated by a gray box which represents mean and standard deviation of twelve parallel replicates.

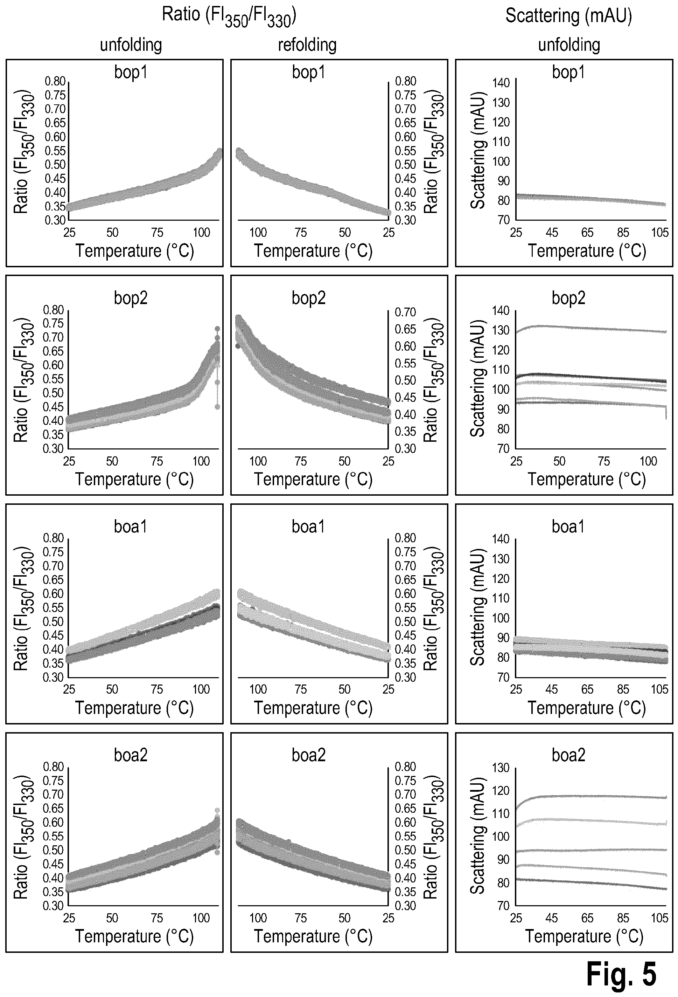

Fig. 5: NanoDSF shows the bifaceted designs to be highly thermostable. Heating (left column) and cooling (middle column) show no unfolding or refolding transitions for all four designs (bop1, bop2, boa1, and boa2) within the temperature range from 25 °C to 110 °C. Ratio values indicate relative fluorescence intensity at 350 nm and 330 nm. Scattering signal during the heating phase (right column) also indicate no aggregation of the designs. At least four (at most seven) technical replicates are shown for each design.

Fig. 6: SPR titrations of the bifaceted designs (bop/boa) compared to the starting template (bv6). SPR sensograms and the corresponding fit of a two-fold dilution series starting at 20 nM of bv6, bop1, bop2, boa1, and boa2. Binding kinetic parameters that were calculated from the displayed fits (Table 2; indicated in bold numbers).

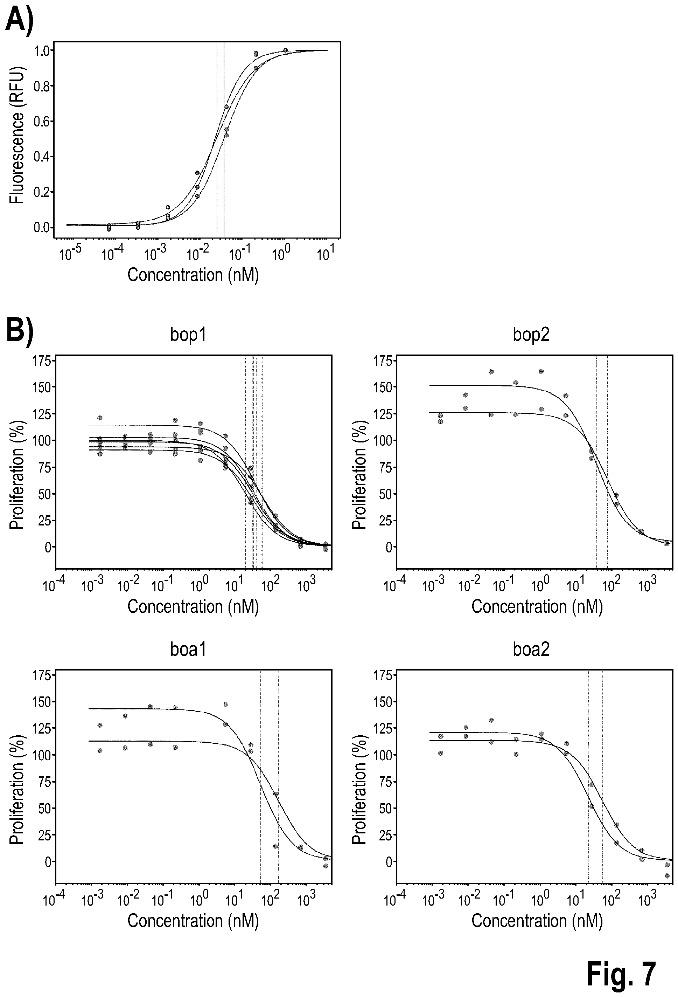

Fig. 7: The bop1 design is the most potently inhibits G-CSF activity with no residual receptor activation. (A) The half-maximal effective concentration ($EC_{50}$) of the bop2 was determined for non-inhibiting concentrations observed in NFS-60 activity assays (cf. Fig. 3F). The fits of three independent experi- ments are shown. Mean and standard deviation values are provided in Table 2. (B) The half-maximal inhibitory concentrations ($IC_{50}$, indicated by dotted lines) were obtained from the corresponding competitive inhibition NFS-60 assay (cf. Fig. 2G) by fitting the individual independent replicates. Mean and standard deviation of the obtained $IC_{50}$ values is presented in Table 2.

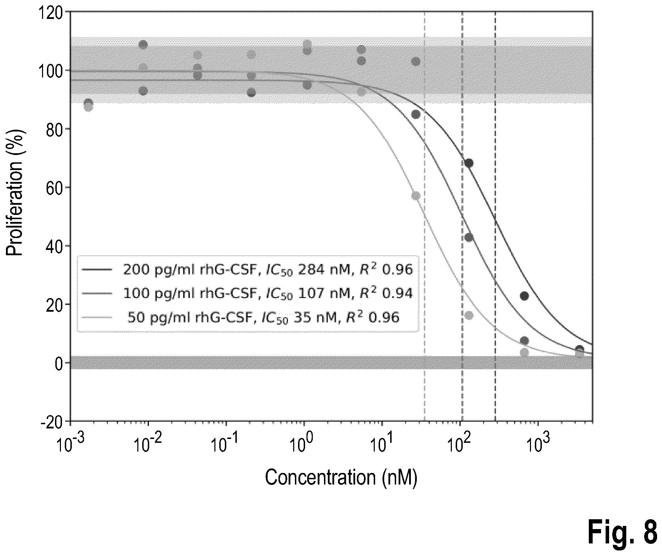

Fig. 8: Bop1 outcompetes G-CSF in a dose-dependent manner. Competitive inhibition assays of NFS-60 proliferation using varying concentrations of bop1 against three concentrations of G-CSF (200, 100, 50 pg/mL). The half-maximal inhibitory concentrations ($IC_{50}$) were obtained from the corresponding fit.

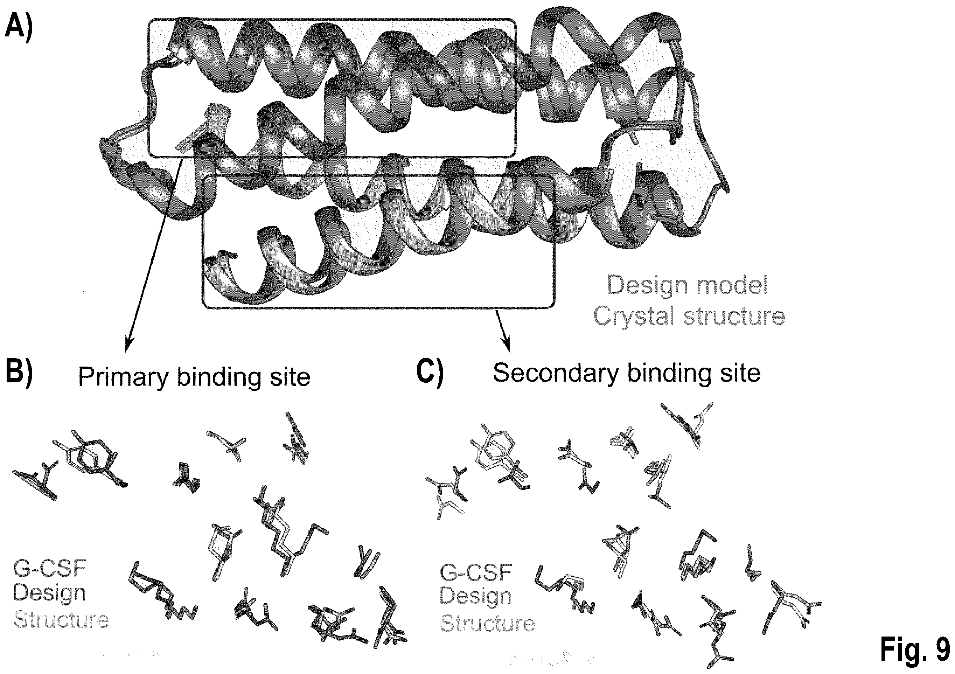

Fig. 9: Crystal structure of bop1 matches its design model at atomic resolution. (A) Alignment of the crystal structure of bop1 (orange) with the design model (teal) shows a global backbone RMSD of 1.03 Å. Primary and secondary binding site helices are colored in purple and gray, respectively. (B, C) Backbone RMSD of the binding sites show the primary binding site of the design model and the crystal structure to align better to the corresponding positions in the G-CSF structure (0.6 Å; PDB: 2D9Q) than the secondary binding site (1.2 Å).

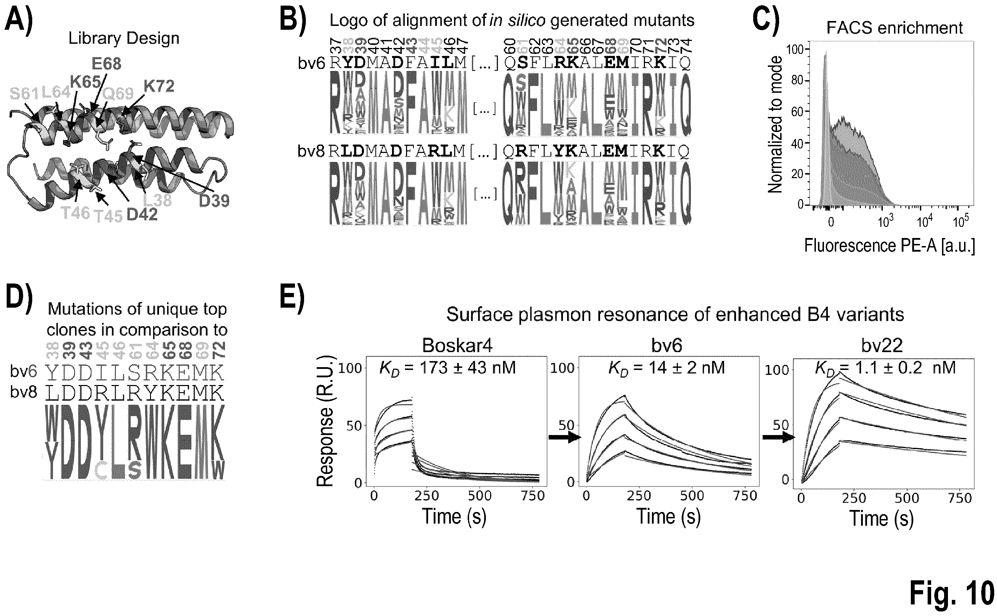

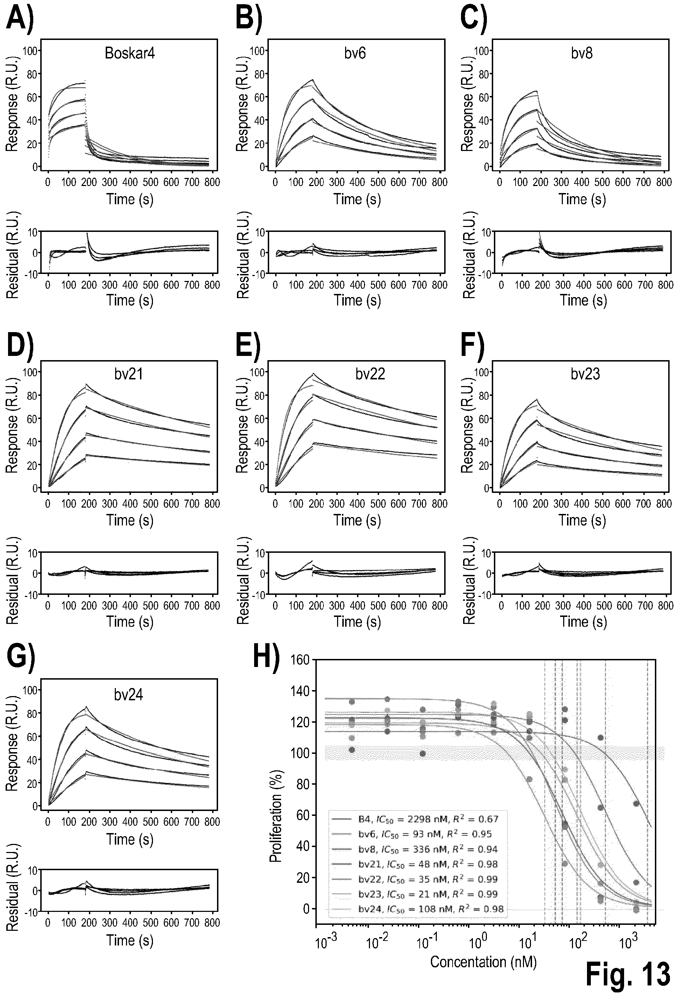

Fig. 10: Computationally-guided affinity enhancement of the primary binding site. (A) Position on the Boskar4 structure subjected to optimization in the modelled complex with G-CSFR. In total 11 residues were optimized (orange or red). These included five positions identified to be critical for G-CSFR binding based on an alanine-scanning study of G-CSF (red). (B) Comparison between the mutants generated by the Damietta combinatorial sampler in a logo that contains the alignment of the mutated sites. (C) High-throughput screening of the bacterial display library resulted in a strong FACS-enrichment of better binding clones. (D) Sequence logo of the top 10 selected clones. Note: The amino acid position marked "43" ("D") should correctly read position "42". (E) SPR measurements of G-CSFR binding to Boskar4 (original design), bv6 (first-generation variant), and bv22 (second-genera- tion variant) demonstrate the enhancement in binding affinity over the first and second round of computationally-guided affinity maturation (Fig. 13 and Table 2).

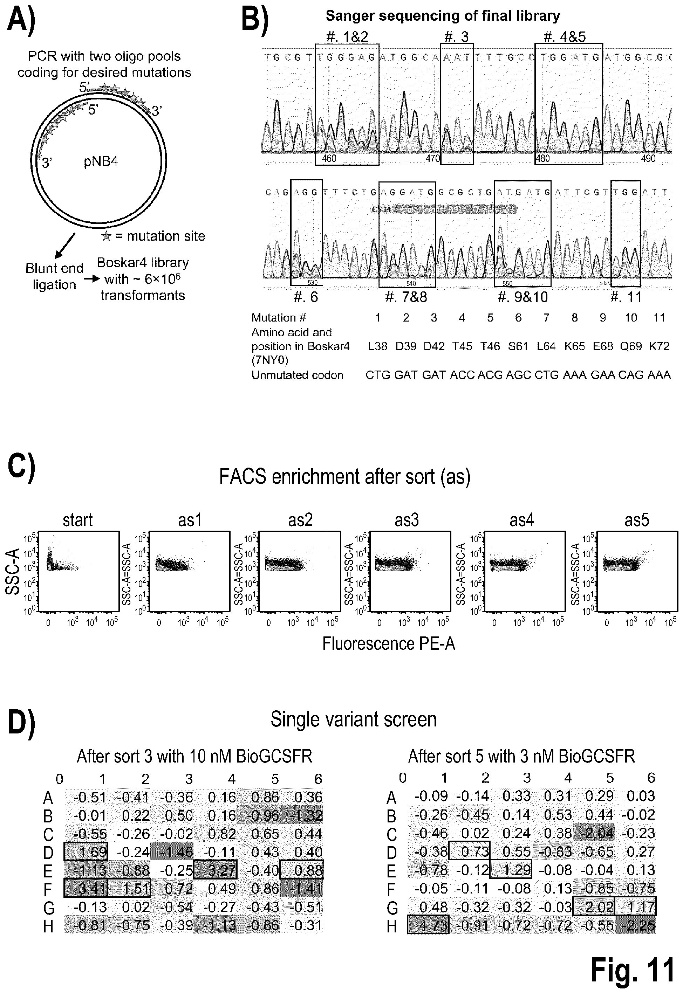

Fig. 11: Library generation and screening. (A) illustration of the PCR mutagenesis strategy used for library generation. (B) Sanger sequencing analysis of mutated sites. (C) FACS enrichment of binding clones after the start and sort 1 to 5 (SSC-A vs. PE-A). (D) Single-variant screen Z-scores to identify top clones in 96-well assay format.

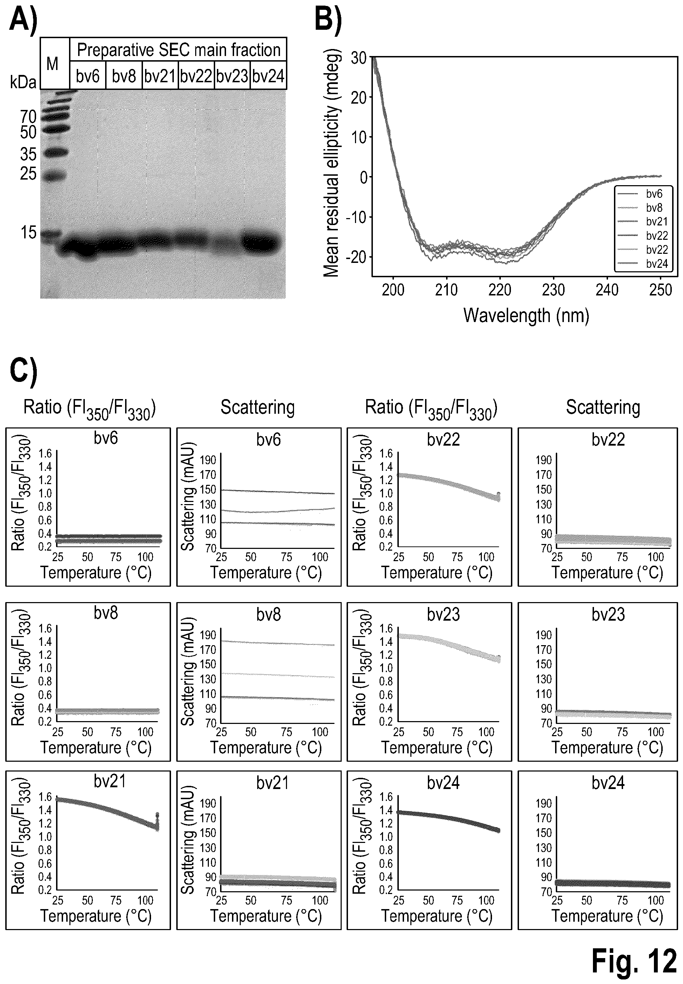

Fig. 12: The enhanced-affinity variants preserve the biophysical properties of the starting designs. (A) SDS-PAGE of main fractions of enhanced-affinity variants bv21, bv22, bv23, and bv24 after preparative size exclusion chromatography. (B) CD analysis of the Boskar4 variants (bv6, bv8, bv21, bv22, bv23 and bv24) show them to be helical. (C) NanoDSF of the variants shows them be to hyper-thermostable. Folding stability and colloidal stability are represented by ratio of fluorescence intensity at 350 nm and 330 nm and the scattering signal, respectively, for at least 4 technical replicates.

Fig. 13: The enhanced variants display stronger receptor-binding affinity and more potent inhibition of G-CSF-induced proliferation. (A-G) SPR sensograms and the corresponding fits of a two-fold dilution series of the Boskar4 (A) and the enhanced Boskar4 variants bv6 (B), bv8 (C), bv21 (D), bv22 (E), bv23 (F) and bv24 (G). The highest concentrations measured were 1250 nM for Boskar4 (B4), 125 nM for bv6 and bv8 and 31 nM for all other shown variants. Binding kinetic parameters derived from the displayed fits are listed in Table 2 (indicated in italic). (H) Competitive inhibition NFS-60 assay with a constant concentration of 50 pg/ml rhG-CSF and the corresponding fit used to derive $IC_{50}$ values for the designs (also compare Table 2).

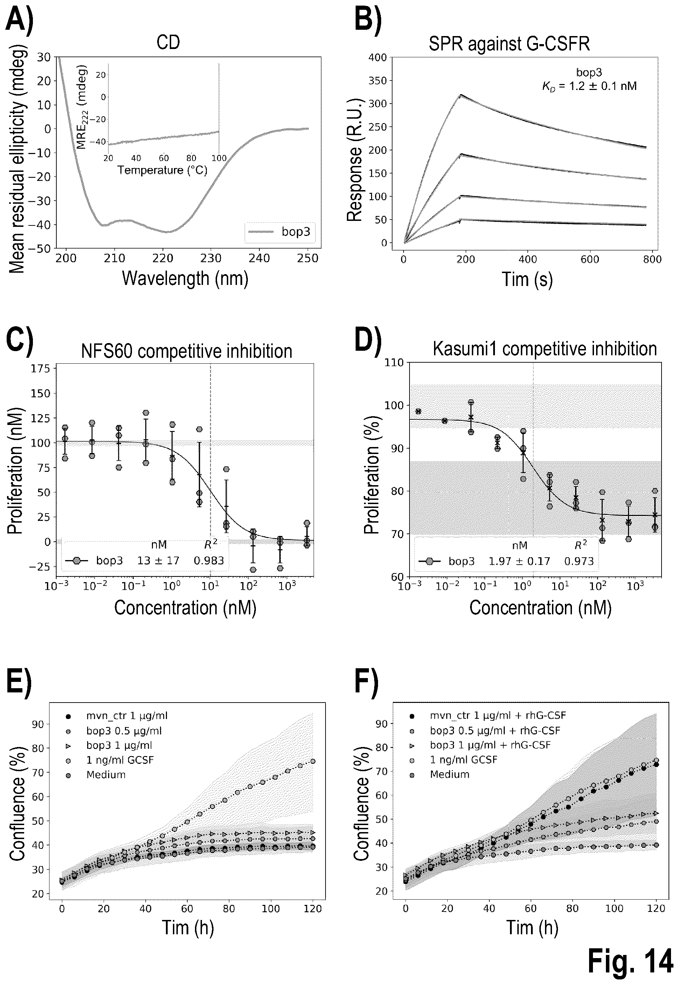

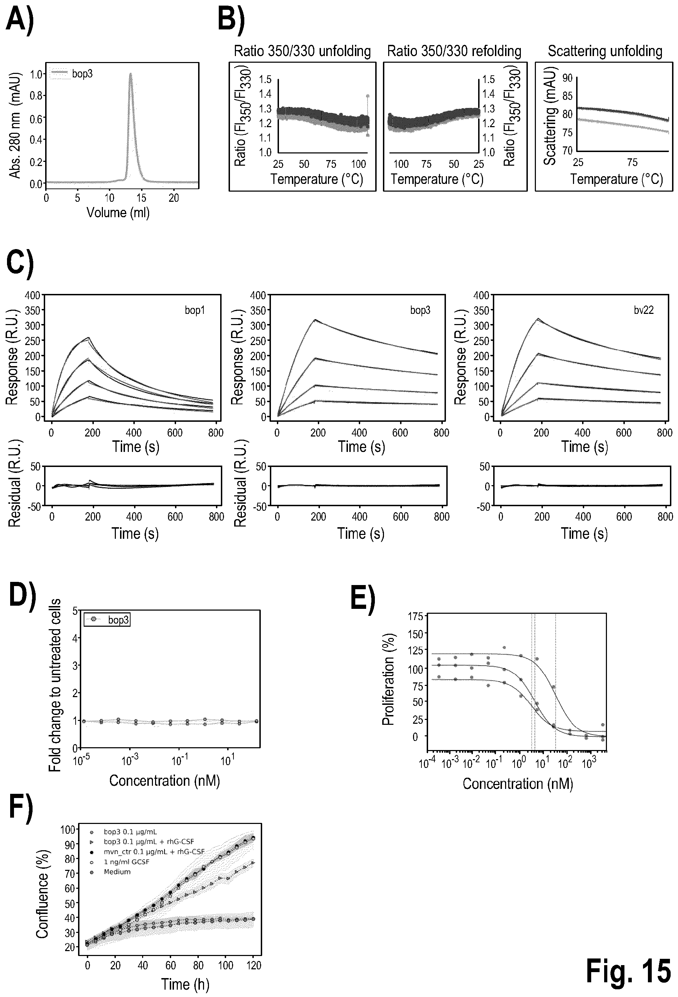

Fig. 14: Characterization of the functionally-enhanced inhibitor bop3. (A) Circular dichroism spectrum and melting curve confirm the helical and hyper-ther- mostable properties of bop3. This thermal stability was also confirmed by nanoDSF (Fig. 15B). (B) SPR measurements also confirm a 10-fold improvement in the receptor-binding affinity. Shown is a twofold dilution series starting from 20 nM of bop3 against chip-immobilized G-CSFR. (C) Competitive inhibition cell assays in NFS-60 cells show bop3 to be approximately 3-fold more potent in inhibiting G-CSF-induced proliferation than bop1, with no observed residual activity (Fig. 15D). (D) Competitive inhibition of G-CSF-induced proliferation in the Kasumi-1 AML cell line using bop3. In both NFS-60 (C) Kasumi-1 cells (D) three independent experiments each, and the half-maximal inhibitory concentrations ($IC_{50}$ in nM) were obtained from a corresponding fit. In both assays, a constant concentration of rhG-CSF was employed, with 50 pg/mL in (C) and 400 pg/mL in (D), each corresponding to 100% proliferation. (E, F) CD34[+] HSPCs proliferation assays using 0.5 μg/ml or 1 μg/ml of bop3 either without (E) or with the addition of 1 ng/ml rhG-CSF (F). Moevan_control (mvn_ctrl) was used as negative control. Shown is mean and standard deviation of two independent replicates, with three technical replicates per each independent replicate.

Fig. 15: Grafting the bv22 binding site as the primary binding site in bop1 yields the most potent inhibition, bop3. (A) The bop3 is exclusively monomeric according to analytical SEC. Chromatogram shown was obtained using Cytiva Superdex 75 Increase 10/300 GL. (B) NanoDSF was performed in a temperature range from 25 °C to 110 °C. Fluorescence intensity ratio at 350 nm and 330 nm, and the scattering signal of 4 technical replicates are shown. (C) SPR sensograms (black lines) and the corresponding fits (green lines) of a two-fold dilution series starting at 20 nM of bop1, bop3 and bv22. Binding kinetic parameters derived from these fits are provided in Table 2 (indicated underlined). (D) NFS-60 activity assay of two independent biological replicates of bop3 show no residual activity. The gray shade indicates the mean and standard deviation of the fold-change of untreated cells to the mean of untreated cells. (E) Competitive inhibition assays of G-CSF-induced proliferation in NFS-60 cells show bop3 to possess lower $IC_{50}$ values (indicated by dotted vertical lines). Results were obtained from a fit of three independent replicates. Mean and standard deviation of the individual fits are provided in Table 2. (F) 0.1 μg/ml of bop3 was tested in a healthy donor's CD34[+] HSPCs proliferation assay either without or with the addition of 1 ng/ml rhG-CSF. 0.1 μg/ml Moe-van_control (mvn_ctrl), a protein lacking a functional G-CSFR binding site, was employed as negative control. Shown is mean and standard deviation of three parallel replicates.

EMBODIMENTS

1. Material and methods

*Modeling of bifaceted variants of G-CSFR-binding designs*

The original Boskar4 template encodes a single G-CSFR-binding site (PDB: 7NY0) that matches site II of the G-CSF (PDB: 2D9Q). This binding site is mounted across the helix 2 and helix 3 (H2/3) regions of the Boskar4 starting template, and is herein referred to as the primary binding site. Boskar4 structure however allows for grafting another binding site on helices 1 and 4 (H1/H4) due to the pseudosymmetry of the 4-helix-bundle, which can constitute a secondary binding site. This additional graft of a secondary binding site on H1/H4 was performed in both parallel and antiparallel orientations with respect to the primary binding site, where an AlphaFold2 model of the affinity-enhanced variant of Boskar4; bv6 was used. The antiparallel grafts; named boa (bivalent-orientation antiparallel) were modelled using either a selection of 12 or 15 binding-site residues, denoted as boa1 and boa2, respectively (Table 1). The parallel orientation grafts, named bop (bivalent-orientation parallel), were modelled using either 12 or 17 binding-site residues, denoted as bop1 and bop2, respectively (Table 1). The grafting position was adjusted to minimize the backbone alignment RMSD of the re-grafted site, where the resulting models and complexes with the receptor were modelled using AlphaFold2. The sequences of the final designs are provided in the sequence listing.

Table 1: Shown are the mutations in bv6 compared to the given design (bop1, bop2, 120 boa1, and boa2) and the corresponding residue in G-CSF (2D9Q).

| bop1 | G-CSF | bop2 | G-CSF | boa1 | G-CSF | boa2 | G-CSF |
|------|-------|------|-------|------|-------|------|-------|
| A5D | D104 | A5D | D104 | A9Q | Q11 | A9Q | Q11 |
| A9Y | L108 | A9Y | L108 | E10S | S12 | E10S | S12 |
| K13D | D112 | E10D | D109 | K13R | L15 | K13R | L15 |
| A16I | T115 | K13D | D112 | G14K | K16 | G14K | K16 |
| E17L | T116 | A16I | T115 | Y18M | Q20 | Y18M | Q20 |
| A20Q | Q119 | E17L | T116 | R21K | K23 | A20R | R22 |
| V101Q | Q11 | A20Q | Q119 | A98D | D104 | R21K | K23 |
| E102S | S12 | R21Q | Q120 | E102Y | L108 | A98D | D104 |
| A105R | L15 | K23E | E122 | Q106D | D112 | E102Y | L108 |
| Q106K | K16 | V101Q | Q11 | A109I | T115 | I103D | D109 |
| A109E | E19 | E102S | S12 | F110L | T116 | Q106D | D112 |
| F110M | Q20 | A105R | L15 | K113Q | Q119 | A109I | T115 |
| | | Q106K | K16 | | | F110L | T116 |
| | | A109E | E19 | | | K113Q | Q119 |
| | | F110M | Q20 | | | Q116E | E122 |

(continued)

| bop1 | G-CSF | bop2 | G-CSF | boa1 | G-CSF | boa2 | G-CSF |
|------|-------|------|-------|------|-------|------|-------|
| | | E112R | R22 | | | | |
| | | I117D | D27 | | | | |

*Protein over-expression and purification*

Synthetic genes of all designs were cloned into a pET28a downstream of N-terminal His-tag and thrombin cleavage site. Expression was performed in Escherichia coli (*E. coli*) BL21(DE3) cells grown in LB medium supplemented with 40 $\mu$g/ml kanamycin in a shaking flask incubator 37 °C and 160 rpm. Cells were induced at an optical density at 600 nm (OD600) of approximately 0.6, using 0.5 mM IPTG, and the expression of the target protein was carried out at 25 °C and 160 rpm for approximately 16 hours. After 16 hours, the cells were harvested by centrifugation at 5000 g for 30 minutes. The supernatant was discarded, and the cell pellet was lysed using ultrasonic homogenization in a lysis buffer containing 50 mM Tris (pH 8), 100 mM NaCl, 20 $\mu$g/ml DNase) (A3778, ITW Reagents), and a protease inhibitor cocktail (04693132001, Roche). The lysate was then centrifugated at 16,000 g for 30 minutes, and the resulting supernatant was loaded onto nickel immobilized metal affinity chromatography (IMAC) beads for batch purification (11912422, Macherey-Nagel). Subsequently, size exclusion chromatography (SEC) was performed on a Superdex® 75 Increase 10/300 GL (29148721, Cytiva) using PBS as the mobile phase. Fractions expected to contain the protein of the desired size were concentrated using ultrafiltration with a 10 kDa cutoff membrane (UFC8010, Amicon® Ultra) and were analyzed again with SEC under the same conditions. Aliquots of these concentrated fractions were frozen at -20 °C until further use.

*Analysis of folding stability*

Circular dichroism (CD) spectra were obtained using a JASCO J-810 spectrometer for protein samples diluted to a concentration of 0.2 mg/ml in PBS buffer at pH 7.0. The CD spectra were recorded by measuring the residual ellipticity of three accumulations, with a resolution of 0.1 nm over a range of 250-190 nm. To determine the melting temperature with CD, the average residual ellipticity was measured at a wavelength of 222 nm over a temperature range of 20 °C to 100 °C with a temperature increase of 0.5 °C per minute. Nanoscale-differential scanning fluorimetry (nanoDSF) experiments were carried out using a Prometheus NT.48 instrument (Nanotemper Technologies) in standard Prometheus capillaries (Nanotemper, PR-C002). These measurements were made on protein samples with a concentration of 1 mg/mL in PBS. The temperature ramp for melting and cooling was set at 1 °C per minute, ramping from 25 °C to 110 °C, followed by a reverse ramp back to 25 °C.

*Binding affinity determination using surface plasmon resonance*

To evaluate the affinity of the designs to G-CSFR, multi-cycle kinetics experiments were conducted using a Biacore X100 system (GE Healthcare Life Sciences). Recombinant human G-CSFR (Glu25-Pro621, 381-GR/CF, R&D Systems) was diluted to a concentration of 50 $\mu$g/mL in a 10 mM acetate buffer at pH 5.0 and immobilized on a CM5 sensor chip (GE Healthcare 29149604) using amine coupling chemistry. The protein samples were diluted in a running buffer consisting of PBS with 0.05% v/v Tween-20. The experiments were carried out at a temperature of 25 °C and a flow rate of 30 $\mu$L/min. The sample solutions were injected sequentially over the functionalized sensor chip surface for 180 s, followed by a dissociation phase of 600 s with running buffer. Four different concentrations for each sample were measured which are indicated in the corresponding legend of the corresponding figure. After each run, the sensor surface was regenerated by injecting a 50 mM NaOH solution for 60 s. To analyze the data, the reference responses and zero-concentration sensograms were subtracted from each dataset (double-referencing). The Biacore X100 Evaluation Software following a 1:1 binding kinetic model was used to determine the association rate ($k_a$), dissociation rate ($k_d$), and equilibrium dissociation constant ($K_D$) constants. Leave-one-out cross-validation was used to determine mean and standard deviation of $k_a$, $k_d$, and $K_D$.

*NFS-60 cell proliferation assay*

The NFS-60 cells were cultured at 5% $CO_2$ and 37 °C in NFS-60-medium, which consisted of RPMI 1640 medium supplemented with 1 mM L-glutamine, 1 mM Na-pyruvate, 10% FCS, 1% Antibiotic-Antimycotic (15240062, Gibco™), and 12.5% KMG-2: 5637-CM (Conditioned Medium from CLS Cell Lines Service). Prior to the proliferation assay, the cells were washed three times with NFS-60-medium without KMG-2. The assay was conducted in black 96-well plates. Each well contained 45,000 cells in a total volume of 150 $\mu$L NFS-60-medium without KMG-2, cultured under maintenance conditions with different concentrations of the protein sample for 48 hours. Following that, 30 $\mu$L of CellTiter-Blue®

Reagent (G808, Promega) was added to each well, and the cells were further incubated for approximately 90 minutes at 5% $CO_2$ and 37 °C. Subsequently, the fluorescence of each well was measured on a plate reader with an excitation wavelength of 560 nm and emission at 590 nm. The half-maximal effective concentration ($EC_{50}$) of each protein sample was determined by fitting the obtained fluorescence values to their corresponding concentrations using a four-parameter sigmoidal function minimized with the Nelder-Mead Simplex algorithm as implemented in the SciPy.

All NFS-60 cell proliferation assays were conducted as described above unless indicated otherwise. In live cell tracking assays, cells were monitored in the IncuCyte S3 Live-Cell Analysis System (Essen Bio) with a 10x objective. Cell proliferation was analyzed using IncuCyte S3 Software. For these experiments NFS-60 cells were maintained at 5% $CO_2$ and 37 °C in NFS-60-medium (RPMI 1640 medium with additional 2 mM L-glutamine, 1 mM Na-pyruvate, 10% FCS, 1% Pen-Strep and 10 ng/mL rhG-CSF). Prior to the proliferation assay, the cells were washed three times with PBS and starved for at least 4 hours in NFS-60 medium without rhG-CSF. The assay was then performed in an L-ornithine pretreated 96-well plate, with 45,000 cells seeded per well.

*NFS-60 competitive inhibition assay*

The NFS-60 cells were cultured under the same condition as described for the NFS-60 cell proliferation assays with the following changes. After washing the cells three times with NFS-60-medium without KMG-2, a final constant concentration of 50 pg/mL of rhG-CSF (unless otherwise indicated in the figures) was applied to the cells together with varying concentrations of the designed proteins. The choice of rhG-CSF concentration mimics the reported physiological serum levels in healthy individuals. Fluorescence measurements were acquired for each experimental condition and subsequently min-max normalized against control samples. These control samples consisted of cells treated with and without the constant concentration of rhG-CSF. From these values the half-maximal inhibitory concentration ($IC_{50}$) was determined in the same way as described for the EC50 parameter in the proliferation assays.

*Structure determination of bop1 using X-ray crystallography*

Crystallization screens were set up with a Mosquito robot (TTP Labtech) in 96-well plates at 21 °C, using 50 $\mu$L of reservoir solution and sitting drops containing 400 nL of reservoir and 400 nL of protein solution of bop1 at a concentration of 9 mg/ml. Within 14-30 days, crystals of bop1 grew in a condition 0.1 M MES pH 7.0, 20 % (w/w) PEG 8,000. Crystals were cryoprotected through the addition of 20% PEG 400, flash cooled and stored in liquid nitrogen until data collection. Diffraction data were collected at 100K on an EIGER 16M detector at beamline X10SA at the Swiss Light Source (SLS). Data were processed and scaled using XDS and the structures solved using molecular replacement with MOLREP and the AF2 model of bop1 as a search model. The structure was completed by cyclic refinement with REFMAC5 and modelling using Coot. Data collection and refinement statistics, coordinates, and structure factors are deposited in the PDB under accession code 8QUP.

*Computationally-guided affinity maturation*

In order to further enhance the binding affinity of the primary binding site of bop1, the inventors selected 11 amino acid positions for computational optimization. The structural models were built as a complex of each of the 17 NMR frames of Boskar4 (7NY0) structurally-aligned to the G-CSF:G-CSFR complex (2D9Q). Two starting template sequences of the ligand were based on the enhanced affinity variants bv6 and bv8 and modelled separately into the complexes using Modeller; yielding a total of 34 complexes. For each of these complexes all 11 positions were mutated in silico to all proteinogenic amino acids (but G, P, and C) utilizing the combinatorial sampler of Damietta (v0.32). The combinatorial design simulations were performed in ten scrambled-order replicas for each modelled complex, resulting into a total of 612 unique designed amino acid sequences. The sequences of the starting variants bv6 and bv8 were added to this pool of unique sequences, resulting in a total of 614 sequences. The obtained amino acid sequences were split into two pools of contiguous fragments, the first encoding the binding protein till L55 (numbering of 168 PDB:7NY0) and the second starting from D56 (number of PDB:7NY0) till the end of the protein sequence. The two amino acid sequence pools were used to design two oligo pools encoding for the corresponding mutants. In total oligo pool 1 covered 298 unique oligos, and oligo pool 2 included 330 unique oligos. These two oligo pools were ordered (IDT, Inc.) encoding the desired mutations on a forward and reverse primer pair to generate a library with a maximum degeneracy and diversity of $9.8 \times 10^4$. Finally, the library was generated by PCR using the two oligo pools to linearize an E. *coli* display system plasmid encoding a fusion between the N-terminal part of intimin and Boskar4 (pNB4, sequence listing). The obtained PCR product was purified with the Wizard® SV Gel and PCR Clean-Up System (A928, Promega) and blunt end ligation was performed followed by electroporation of freshly made competent E. *coli* DH10BT1R cells to yield $6.0 \times 10^6$ total transformants.

In more detail, prior to transformation in DH10BT1R cells, 0.4 U/$\mu$L T4 polynucleotide kinase was incubated with 5 ng/$\mu$L of linearize PCR product for 30 min at 37 °C, according to manufacturer instructions. After that, 0.25 U/$\mu$L T4 DNA

ligase was added and incubated overnight at 16 °C. The ligation product was purified with the PCR Clean-Up System (A928, Promega) and eluted into 20 µL filtered ddH$_2$O. The complete 20 µL were transformed into electrocompetent *E. coli* as described. Specifically, 50 mL of freshly grown E. coli (DH10B T1R) of an OD600 of ~0.6 was washed two times with 35 mL filtered ddH$_2$O. After washing, the bacterial pellet was resuspended with the purified ligation mix. The electroporation was performed at 1250 V and 5 ms. The cells were grown in 1 mL SOC Outgrowth Medium (B9020, NEB) for 2 h in a standard glass tube at 30 °C with 160 rpm. A 10-fold dilution series of the cells were made in SOC-medium and they were plated on agar plates containing 34 µg/mL chloramphenicol and 2% (w/v) D-glucose to estimate the total number of transformants. The rest of the sample was plated on five agar plates of the same type and incubated at 30 °C for about 20 h. The bacterial lawn was scraped from the plates and well mixed in 10 ml LB-medium. The plasmids were isolated from 500 µL of bacterial suspension and the obtained plasmid library was analyzed by sanger sequencing. Single Boskar4 variant genes was amplified by PCR from the pN vector and ligated between Ndel and Xhol replacing Boskar4 of pET28a.

To perform cell sorting of the bacterial clones bound to G-CSFR, fluorescently-labelled rhG-CSFR was prepared. Specifically, a total of 100 µg of recombinant human G-CSFR (381-GR/CF, R&D Systems) was subjected to a reaction with Biotin-NHS (H1759, Sigma) in a 1:20 molar ratio (G-CSFR:Biotin-NHS), within a 950 µL solution of PBS. The mixture was incubated at room temperature (RT) for a duration of 2 hours. Subsequently, the reaction was quenched by adding 50 µL of Tris (1 M pH 7.5). The resulting product underwent purification through a desalting column (Sephadex G25 PD-10, GE Healthcare), followed by concentration of elution fractions via ultrafiltration (3 kDa, UFC800324, Merck). The biotinylated rhG-CSFR (BioG-CSFR) was then aliquoted and stored at -20 °C until it was utilized for further experiments.

The screening process of the library also followed a modified protocol. Initially, DH10BT1R *E. coli* cells were transformed using the display vector containing the protein library. The bacteria were grown on LB agar plates with 34 µg/mL chloramphenicol and 2% (w/v) D-glucose at 30 °C. After harvesting the plates, 1 mL of resuspended bacteria with an OD600 of 3.3 was washed twice with 1 mL LB and then diluted to a starting OD600 of 0.33 in 10 ml LB with 34 pg/mL chloramphenicol. The cells were grown at 30 °C and 160 rpm and after 1 hour, 50 µM IPTG was added to the culture, and it was further grown for 3 hours under the same conditions. Cells were collected (equal to 1 mL of a cell resuspension with an OD600 of 1) were washed twice with 1 mL PBS and resuspended in 400 µL PBS. In the next step, 190 µL of this cell suspension was incubated with 10 nM BioG-CSFR at room temperature for 1 hour, followed by a single wash with 1 mL PBS. The washed cell pellet was resuspended in in 200 µL PBS, to which 0.375 µL PE-Strepta-vidin (405203, BioLegend) was added, and incubated at 4 °C for 30 minutes. Finally, the sample was washed once more with 1 mL PBS and resuspended in 1 mL PBS. The screening was performed using a BD FACSMelody™ Cell Sorter having set the PTM voltage for PE to 517 V, SSC to 426 V and an SSC threshold of 673 V. No gating was applied, and 100,000 events were measured per sample. For sorting, 300 µL of the sample was mixed with 4 mL ice-cold PBS, and the flow rate was adjusted to achieve an event rate of approximately 8000 events/sec. Approximately, the top 0.1% of the total population, based on an SSC-A/PE-A plot, was sorted into a 1.5 mL tube containing 200 µL LB medium, which was cooled to 5 °C. The sorting process continued until at least 20 times more events were screened than the complexity of the corresponding library. All sorted cells were plated on LB agar plates with 34 µg/ml chloramphenicol and 2% (w/v) D-glucose and were incubated at 30 °C for around 20 hours. The harvested plates were then grown overnight in LB medium with 34 µg/mL chloramphenicol and 2% (w/v) D-glucose at 30 °C under static conditions. From this culture, 1 mL of resuspended bacteria with an OD600 of 3.3 was used for the next selection cycle performed in the same way, and repeated for five iterative cycles. All flow cytometry results were analyzed using FlowJo™ v10.1 Software (BD Life Sciences).

Single colonies of bacteria pools which were enriched for 3 or 5 cycles of FACS (as described above) were grown overnight in 100 µL LB (supplemented with 34 µg/mL chloramphenicol and 50 µM IPTG) in a 96-well plate sealed with Breathe-Easy® sealing membrane (Z380059, Sigma-Aldrich) at 30 °C with 1100 rpm on a benchtop shaker (Thermomixer comfort, Eppendorf) covered with aluminum foil. At the next day the cells were centrifugated at 3200 g for 5 min, resuspended in 150 µL PBS and centrifugated again. The washing was repeated one more time and the cells got resuspend in 50 µL PBS containing 10 nM BioGCSFR. After the plate was incubated for 1 h at room temperature, the cells were washed one time with 200 µL PBS and resuspended in 50 µL PBS. 25 µL of the resuspended cells were mixed with 25 µL PBS containing 1.25 µg/mL Avidin-HRP conjugate (Invitrogen, 434423) followed by a 30 min incubation at 4 °C in the dark. Afterwards the cells were washed one time with 200 µL PBS and resuspended in 100 µL PBS. The cell density (OD600) of each well was measured with the plate reader. In a separate dark plate (655097, Greiner Bio One) 12.5 µL cell suspension was added to 75 µL PBS. Then 12.5 µL ECL-substrate-solution (1705061, Bio-Rad) was pipetted quickly into each well, the plate was sealed with a parafilm and mixed vigorously for 10 seconds on a vortex orbital shaker. Then the luminescence of each well was measured on the plate reader. The luminescence signal of each variant in each well was analyzed by normalizing to the corresponding cell density and calculating a Z-score for each sample over all wells of the same condition. From the top 5 variants of each condition plasmids were isolated with a plasmid preparation kit (740588, Macherey-Nagel) from a 3 ml overnight culture grown at 30 °C with 160 rpm in LB with 34 µg/mL chloramphenicol and 2% (w/v) D-glucose. Afterwards, the acquired plasmids were sequenced, and distinct Boskar4 variant (bv) genes were identified which were designated as bv21, bv22, bv23, and bv24. The Alignment consensus logos of the mutated sites were illustrated using AlignmentViewer.

*Kasumi-1 competitive inhibition assay*

Kasumi-1 cells were cultured at 5% $CO_2$ and 37 °C in RPMI 1640 medium supplemented with 1 mM L-glutamine, 10% FCS, and 1% Antibiotic-Antimycotic (15240062, Gibco™). In a black 96-well plate, 40,000 cells were seeded in each well with a constant concentration of 400 pg/mL of rhG-CSF, alongside varying concentrations of the designed proteins. The cells were then incubated under standard conditions for 72 hours. CellTiter-Blue® Reagent was added into each well, and the resulting fluorescence was measured as described in the NFS-60 cell proliferation assay. The fluorescence values obtained were subsequently normalized to the cells treated with 400 pg/mL of rhG-CSF. Finally, the $IC_{50}$ was determined as described above. Leave-one-out cross-validation was used to calculate the mean and standard deviation of the $IC_{50}$ across the three biological replicates. This approach was chosen because the biological replicates encompassed varying numbers of treatments.

*Evaluation of dose-dependent effects of inhibitors on the proliferation of CD34+ HSPCs*

Human CD34+ cells were isolated from the bone marrow mononuclear cell fraction of two healthy donors by the Ficoll density gradient centrifugation with subsequent magnetic bead separation using the Human CD34 Progenitor Cell Isolation Kit (Miltenyi Biotech Germany; #130-046-703). Isolated cells were frozen till the experimentation. After thawing, CD34+ cells were cultured for up to five days in Stemline II Hematopoietic Stem Cell Expansion medium (Sigma Aldrich; #50192) supplemented with 10% FBS, 1% penicillin/streptomycin, 1% L-glutamine and 20 ng/ml IL-3, 20 ng/mL IL-6, 20 ng/mL TPO, 50 ng/mL SCF, and 50 ng/mL FLT-3L. Before experimentation, cells were washed twice in ice-cold PBS and 2 × 10⁴ cells/well were incubated in poly-L-lysine-coated 96-well plates for five days in an IncuCyte S3 Live-Cell Analysis System (Essen Bio) with a 10x objective at 37°C and 5% $CO_2$. Cells were incubated in the same Stemline II Hematopoietic Stem Cell Expansion medium supplemented with 10% FBS, 1% penicillin/streptomycin, 1% L-glutamine and 1 ng/mL of rhG-CSF or PBS in the presence or absence of designed inhibitors (i.e. bop1 or bop3) or an inert helical protein (moevan_control), at concentrations ranging from 5 μg/mL to 0.1 μg/mL (as indicated in the respective figure). Cell proliferation was analyzed using IncuCyte S3 Software. All experiments in this study involving humanderived HSCPs samples were conducted according to Helsinki's declaration, and study approval was obtained from the Ethical Review Board of the Medical Faculty, University of Tübingen (012/2021BO2).

2. Results

*Design of single-domain, bifaceted G-CSFR binders*

In this study the inventors used Boskar4 as their starting design template to build G-CSFR inhibitors. Boskar4 is a de novo designed protein domain that is small (13 kDa), proteolytically and thermally stable, and binds to G-CSFR at the same binding site of the native ligand, G-CSF. Previously the inventors showed potent G-CSFR agonists can be made by tandemly-repeated Boskar4 fusions, which dimerize and activate the receptor. Through this work the inventors pursue the inverse goal of creating potent G-CSFR inhibitors based on single-domain Boskar4 template. Based on the inventors' previous work to create rigid Boskar4-based dimerizers that can associate G-CSFR in non-native geometries the inventors observed that the inter-transmembrane domain (inter-TMD) spacing of the receptor subunits to be a critical parameter affecting activity. Specifically, ligands that impose 2:1 receptor:ligand complexes with longer inter-TMD spacing result in weaker activity and reduced receptor dimerization. However, an additional challenge to this end relates to identifying Boskar4 variants that are exclusively monomeric in solution to avoid any unintended oligomerization, and thus unintended activation, of the receptor. Accordingly, the inventors' hypothesis to create inhibitors aimed to exploit the concept of creating a rigid, single-domain, 96 bivalent receptor binder that associates G-CSFR in an inactive configuration (Fig. 1A). Moreover, the inventors also initially hypothesized that the ligand self-interactions could potentially be reduced by repurposing more of the ligand's surface area for receptor binding. The up-down four-helix-bundle of Boskar4 has a cuboidlike regularity, where only one facet of the cuboid encodes the receptor binding site (herein referred to as the primary binding site). Given the pseudosymmetry of the helical bundle, the opposite facet of the bundle can be used as template to graft another secondary binding site that can be juxtaposed in a parallel or an antiparallel orientation relative the primary binding site. Modelling the resulting receptor:ligand 2:1 complexes indicated the antiparallel configuration to result in large inter-TMD spacing (292 Å), this compares with 60 Å for the native G-CSF:G-CSFR complex. Such antiparallel spacing would yield in a highly strained complex in the physiological context of membrane-embedded receptor chains (Fig. 1B). Conversely, the inventors' models predicted the parallel configuration to be signaling-competent given its relatively short inter-TMD spacing (188 Å), as compared to the modelling of receptor geometries induced by G-CSF or the inventors' previously described geometric modulators.

To start from a near-optimal design template, the inventors used a structural model of bv6. The bv6 is a functionally-enhanced variant (5-point mutant) of Boskar4 that binds 12-fold tighter to G-CSFR, and functions as a more potent granulopoietic agonist when tandemly repeated in a two-domain fusion.

Whereas the primary binding site lies on H2 and H3 (i.e. 2nd and 3rd helix of bv6), the secondary binding site was grafted onto H1 and H4 (i.e. 1st and 4th helix of bv6) in antiparallel orientation to the primary site. The inventors grafted two sets of residues representing a small patch (12 grafted residues) and a large patch (15 grafted residues) of the primary binding site. The final binding sites of corresponding AlphaFold2 models of each design matched the original binding site within 1.13 Å and 1.18 Å backbone atoms root mean square deviation (RMSD) for the small and large grafts, respectively. The inventors refer to this class of designs as boa (bivalent-orientation antiparallel), where boa1 or boa2 refer to the designs with either the small or the large secondary binding patches, respectively.

In order to conversely test the inventors' hypothesis that the boa designs yield non-activating G-CSFR complexes, the inventors sought to generate control designs with parallel orientation of binding sites based on bv6 as well; bop (bivalent-orientation parallel). Likewise, the secondary binding site was grafted from either a small patch (bop1, 12 residues) or a large patch (bop2, 17 residues) from the primary binding sites. Akin to the boa designs, the grafted binding sites of corresponding AlphaFold2 models of bop1 and bop2 were compared to the intended biding site and exhibited 1.17 Å and 1.50 Å backbone atoms RMSD. The backbone RMSD of all designs to the original binding site was on average 0.57 $\pm$ 0.08 Å for the primary binding site and 1.24 $\pm$ 0.17 Å for the secondary binding site (Fig. 2). Therefore, the inventors expected the secondary binding site to bind G-CSFR less efficiently than the primary binding site. The inventors hence started by experimentally characterizing these four designs (boa1, boa2, bop1 and bop2).

*The bifaceted designs are hyper-stable and exclusively monomeric in solution*

The expression of all four designs in *E. coli* resulted in a high soluble protein yield, which was readily purified to homogeneity using IMAC and preparative SEC. Analytical SEC showed the purified proteins (bop1/2 and boa1/2) to be strictly monomeric in solution (Fig. 3A, B). This soluble, monomeric property is advantageous for obtaining inhibitors that do not inadvertently aggregate or oligomerize the G-CSFR, which might lead to receptor activation. In contrast, the single-domain form of the original design, Boskar4, partitions between monomer and dimer in solution (Fig. 4B), where its isolated monomeric fraction could only weakly inhibit G-CSFR activation (Fig. 4C, D). The starting template in this study, bv6, also showed a tendency to form dimers in solution, similar to Boskar4 (Fig. 3C), which explains its residual capacity to activate G-CSFR (Fig. 4E).

CD measurements revealed strong $\alpha$-helical profiles for all designs indicating their well-folded nature (Fig. 3D). Additionally, nanoDSF showed all designs to be hyper-thermostable. During the temperature ramp none of the designs showed increased scattering up to 110 °C, highlighting the colloidal stability of the proteins (Fig. 5). For boa1 and boa2, the fluorescence ratio (350 and 330 nm) was unchanged during the heating ramp, while for bop1 and bop2 it indicated a melting onset beyond 90 °C. However, the cooling ramp of bop1 and bop2 (from 110 °C to 25 °C) showed this melting signal onset to be reversible.

*The designs show strong binding and inhibition of G-CSFR*

The inventors then set out to evaluate the binding affinities of the bifaceted designs to G-CSFR using surface plasmon resonance, which was done in a direct comparison to their starting template bv6. The results revealed a pattern whereby all the bivalent designs were more affine than the starting template, and the large-patch grafts (boa2 and bop2) bound G-CSFR tighter in comparison to the small-patch grafts (boa1 and bop1). Specifically, bv6 showed the weakest binding affinity (KD = 4.3 $\pm$ 0.4 nM) in comparison to the other designs (Fig. 3E, 6 and Table 2), which points to the putative role of the secondary binding site in reducing the apparent dissociation rate ($k_d$).

Table 2: The estimated dissociation rate ($k_d$), association rate ($k_a$), and dissociation constant ($K_D$) of the corresponding designs are provided the corresponding colors signify whether the samples were measured in the same experiments. SPR parameter for Boskar4 and bv6 colored in green were adopted. Furthermore, the half-maximal inhibitory concentration ($IC_{50}$) and the half-maximal effective concentration ($EC_{50}$) of NFS-60 assays are presented, along with indications of residual activity for non-inhibiting concentrations.

| Design | SPR parameter | | | | $IC_{50}$ mean $\pm$ sd (nM) | $EC_{50}$ mean $\pm$ sd (pM) | Residual activity |
|---|---|---|---|---|---|---|---|
| | $k_a$ (M$^{-1}$s$^{-1}$) | $k_d$ (s$^{-1}$) | $K_D$ (M) | Chi$^2$ (RU$^2$) | | | |
| bv6 | (1.2 $\pm$ 0.4) $\times$ 10$^6$ | (5.1 $\pm$ 1.1) $\times$ 10$^{-3}$ | (4.3 $\pm$ 0.4) $\times$ 10$^{-9}$ | 2.33 | 93.1 | NA | yes |
| | (2.4 $\pm$ 0.7) $\times$ 10$^5$ | (3.3 $\pm$ 0.4) $\times$ 10$^{-3}$ | (1.4 $\pm$ 0.2) $\times$ 10$^{-8}$ | 2.51 | | | |

(continued)

| Design | SPR parameter | | | Chi$^2$ (RU$^2$) | $IC_{50}$ mean $\pm$ sd (nM) | $EC_{50}$ mean $\pm$ sd (pM) | Residual activity |
| --- | --- | --- | --- | --- | --- | --- | --- |
| | $k_a$ (M$^{-1}$s$^{-1}$) | $k_d$ (s$^{-1}$) | **$K_D$ (M)** | | | | |
| bop1 | **(8.8 $\pm$ 4.0) $\times$ 10$^5$** | **(2.2 $\pm$ 0.6) $\times$ 10$^{-3}$** | **(2.7 $\pm$ 0.4) $\times$ 10$^{-9}$** | **4.76** | 35.3 $\pm$ 12.9 | NA | no |
| | (2.0 $\pm$ 0.6) $\times$ 10$^6$ | (6.3 $\pm$ 0.7) $\times$ 10$^{-3}$ | (3.2 $\pm$ 0.5) $\times$ 10$^{-9}$ | 13.2 | | | |
| bop2 | **(1.9 $\pm$1.1) $\times$ 10$^6$** | **(1.3 $\pm$ 0.3) $\times$ 10$^{-3}$** | **(8.5 $\pm$ 3.2) $\times$ 10$^{-10}$** | **12.7** | 58.2 $\pm$ 28.8 | 28.9 $\pm$ 7.9 | yes |
| boa1 | **(6.9 $\pm$ 1.1) $\times$ 10$^5$** | **(2.4 $\pm$ 0.2) $\times$ 10$^{-3}$** | **(3.7 $\pm$ 0.8) $\times$ 10$^{-9}$** | **2.18** | 106.5 $\pm$ 78.0 | NA | yes |
| boa2 | **(1.4 $\pm$ 0.7) $\times$ 10$^6$** | **(1.3 $\pm$ 0.2) $\times$ 10$^{-3}$** | **(1.1 $\pm$ 0.4) $\times$ 10$^{-9}$** | **12.3** | 37.8 $\pm$ 22.9 | NA | yes |
| bop3 | (1.5 $\pm$ 0.3) $\times$ 10$^6$ | (1.8$\pm$ 0.3) $\times$ 10$^{-3}$ | (1.2 $\pm$ 0.1) $\times$ 10$^9$ | 2.1 | 13.4 $\pm$ 16.9 | NA | yes |
| Boskar4 | *(3.4 $\pm$ 1.7) $\times$ 10$^4$* | *(5.4 $\pm$ 1.4) $\times$ 10$^{-3}$* | *(1.7 $\pm$ 0.4) $\times$ 10$^{-7}$* | *13.9* | 2298.2 | NA | yes |
| bv8 | *(7.2 $\pm$ 6.7) $\times$ 10$^5$* | *(1.7 $\pm$ 1.4) $\times$ 10$^{-2}$* | *(2.6 $\pm$ 0.3) $\times$ 10$^{-8}$* | *5.52* | 336.1 | NA | yes |
| bv21 | *(8.2 $\pm$ 1.4) $\times$ 10$^5$* | *(1.1 $\pm$ 0.1) $\times$ 10$^{-3}$* | *(1.4 $\pm$ 0.1) $\times$ 10$^{-9}$* | *1.34* | 47.8 | NA | yes |
| bv22 | *(8.8 $\pm$ 2.2) $\times$ 10$^5$* | *(9.2 $\pm$ 0.7) $\times$ 10$^{-4}$* | *(1.1 $\pm$0.2) $\times$ 10$^{-9}$* | *3.88* | 35.0 | NA | yes |
| | (1.4 $\pm$ 0.1) $\times$ 10$^6$ | (1.8 $\pm$ 0.1) $\times$ 10$^{-3}$ | (1.3$\pm$ 0.1) $\times$ 10$^{-9}$ | 3.34 | | | |
| bv23 | *(8.6 $\pm$ 1.9) $\times$ 10$^5$* | *(1.5 $\pm$ 0.1) $\times$ 10$^{-3}$* | *(1.8 $\pm$ 0.2) $\times$ 10$^{-9}$* | *1.29* | 21.4 | NA | yes |
| bv24 | *(1.0 $\pm$ 0.4) $\times$ 10$^6$* | *(1.7 $\pm$ 0.3) x 10$^{-3}$* | *(1.7 $\pm$ 0.3) x 10$^{-9}$* | *2.86* | 108.4 | NA | yes |

Regardless of the orientation of the secondary binding site, its small-patch variants, boa1 ($K_D$ = 3.7 $\pm$ 0.8 nM) and bop1 ($K_D$ = 2.7 $\pm$ 0.4 nM), displayed weaker receptor-binding affinity than their large-patch counterparts; boa2 ($K_D$ = 1.1 $\pm$ 0.4 nM) and bop2 ($K_D$ = 0.9 $\pm$ 0.3 nM). These results highlight the symmetric regularity of the template helical bundle, whereby the orientation of the secondary of binding site was less important than the graft size in improving the interaction with the receptor.

To evaluate how the differences in geometry and affinity affect the biological activity of the different designs, the inventors started by testing the proliferative potential of them in a G-CSF-responsive cell line (NFS-60). As expected, these experiments showed the strongest proliferation activator was the design with the large, parallel secondary binding site (i.e. bop2). Also, expectedly, the designs with the antiparallel secondary binding site induced only marginal proliferation increase at a concentration range between 5 and 10 nM concentrations. Interestingly however, the design that showed no measurable residual activity was the one with the small, parallel secondary binding site (bop1) (Fig. 3F). These results highlight that the most efficient receptor dimerizer and activator *in vitro* is indeed is bop2 ($EC_{50}$ = 28.9 $\pm$ 7.9 pM; Fig. 7A). This is in line with the inventors' previous observation from a geometry-rigging design, ori1, which dimerizes G-CSFR at a similar inter-TMD spacing (i.e. dori1 = 206 Å, while dbop2 = 188 Å). Nonetheless, bop1 did not result in any receptor dimerization, and thus activity, under the same conditions.

To evaluate the inhibitory potential of the inventors' designs, the inventors followed up by conducting competitive inhibition assays of rhG-CSF in NFS-60 cells (Materials and methods). At high concentrations, approximately above 10 > nM, all designs showed substantial inhibition of rhG-CSF-induced proliferation. Lower concentrations however showed different levels of design-induced proliferation by bop2, boa1, and boa2, which was not the case for bop1 (Fig. 3G, Fig. 7B). Indeed, bop1 treatment resulted in no residual activation at low concentrations, and possessed the highest inhibitory potency ($IC_{50}$ = 35.3 $\pm$ 12.9 nM; n=6). To validate the specificity of this inhibitory activity, the inventors evaluated the ability of bop1 to outcompete higher concentrations of rhG-CSF in a concentration-dependent manner, which was true for above-physiological concentrations (100 and 200 pg/mL) of G-CSF (Fig. 8). Moreover, the inventors conducted proliferation assays of CD34+ hematopoietic stem and progenitor cells (HSPCs) to assess the biological activity of bop1 on primary human cells. As expected, there was no observable proliferative activity when the cells were treated with up to 1 µg/mL bop1 (Fig. 3H). Conversely, an inhibition of HSPCs stimulated with 1 ng/mL rhG-CSF and treated with 1 µg/mL bop1 was evident (Fig. 3I). Therefore, the inventors selected bop1 as the design candidate to further analyze it and enhance its activity.

*The structure of bop 1 matches the design model at atomic accuracy*

To judge the design accuracy, the inventors sought to solve the crystal structure of the strongest identified inhibitor, bop1. The inventors obtained crystals diffracting to 1.3 Å, with one copy of bop1 in the asymmetric unit, which could be solved by molecular replacement using an AlphaFold2-generated design model (data not shown). Overall, the crystal structure matched the design model to atomic accuracy, with a global backbone Cα RMSD between the structure and the model of 1.03 Å (Fig. 9A). The comparison between the primary binding site residues of bop1 and the native receptor-binding site II of G-CSF (PDB: 2D9Q) showed only minor backbone Cα RMSD of 0.6 Å (Fig. 9B). The alignment was worse however when comparing the secondary binding site residues of bop1 to site II of G-CSF, which yielded and RMSD of 1.2 Å (Fig. 9C). While these results emphasize the weaker structural match, as was expected from the design model, the crystal structure showed no unexpected conformational deformities. This leads us to hypothesize the inability of bop1 to dimerize G-CSFR in cells might be attributed to weak or insufficient receptor interactions through the secondary binding site.

Taken together with the excellent agreement of the bop1 design model and its experimental structure, as well as its superior stability, solubility, and monomeric nature, the inventors were motivated to further improve its inhibitory potency.

*Computationally-guided enhancement of bop1 inhibitory potency*

To improve the inhibitory potency of bop1, the inventors reasoned that enhancing the affinity of only the primary binding site of bop1 can result in stronger G-CSF out-competition, without altering the monomeric nature of bop1, and thus its inability to dimerize G-CSFR. The inventors performed combinatorial design on 11 selected amino acid positions on surface of Boskar4 variants (bv6 and bv8) as mutable and 24 repackable positions across the modelled Boskar4:G-CSFR binding interface, using the Damietta software (Fig. 10A). The inventors then created a library from the sequence profile of lowest energy designs (Fig. 10B, 11A, B; Materials and methods). The resulting library of binders was screened using a bacterial display system (Fig. 10C, 11C; Materials and methods). Five sequential cycles of fluorescence-activated cell sorting (FACS) enriched the bacterial clones displaying binders to fluorescently-labelled rhG-CSFR. After the third and fifth sorts, 48 single clones per condition were screened on plate-based assays to identify the clones with the tightest relative binding signal (Fig. 11D). From the 96 analyzed clones, the top 10 clones were sequenced. The inventors identified four unique enhanced Boskar4 variants, named bv21, bv22, bv23 and bv24. Within these variants the inventors observed that 7 mutated positions have reverted to the starting sequence, indicating the near-optimal sequence of the primary binding site of the bv6 and bv8 variants (Fig. 10D).

The new unique variants bv21, bv22, bv23, and bv24 encoding only the primary binding site (sequence listing) were expressed, purified, and biophysically characterized. All these variants were readily produced, well-folded and highly thermostable (Fig. 12). Receptor-binding assays performed to compare the binding affinity of Boskar4, bv6, bv8, and the new variants (i.e. bv21-24), showed all the new variants to possess higher affinity. Specifically, all the new variants possessed a dissociation constant ($K_D$) within the range of 1 nM to 2 nM, compared with $14 \pm 2$ nM for bv6, $26 \pm 3$ nM for bv8, and $173 \pm 43$ nM for the original Boskar4. Notably, bv22 demonstrated the highest affinity among the variants with a KD of $1.1 \pm 0.2$ nM (Fig. 10E, 13 and Table 2). In their existing form (i.e. encoding only the primary binding site) the new variants already exhibited stronger G-CSFR inhibition in competitive inhibition assays. For instance, bv22 had an $IC_{50}$ of 35 nM, compared to 93 nM for bv6, and 2.3 µM for Boskar4. As observed previously, all tested designs, except for bop1, displayed residual activity at non-inhibiting concentrations (Fig. 13H).

Finally, in order to combine the potent inhibitory activity of bv22 and the eliminated residual activity of bop1 the inventors created the bop3 design, which encodes the primary binding site of bv22 and the secondary binding site of bop1. The bop3 design was readily produced and purified, well-folded and hyper-thermostable (Fig. 14A, 15A, B). SPR titrations of bop3 showed it to bind G-CSFR with a $K_D$ of $1.2 \pm 0.1$ nM (Fig. 14B), which is comparable to that observed for bv22 ($K_D = 1.3 \pm 0.1$ nM; Fig. 15C). As expected, the proliferation assays in NFS-60 cells indicated no proliferative activity for bop3 across a broad range of concentrations (Fig. 15D). Competitive inhibition assays in the same cells showed bop3 to exhibit 3-fold improvement in inhibitory potency compared bop1 ($IC_{50} = 13.4 \pm 16.9$ nM; Fig. 14C and 15E). The inventors hence further evaluated the potential of bop3 to inhibit a G-CSF-dependent acute myeloid leukemia (AML) cell line, Kasumi-1.

The results of this competitive inhibition assay G-CSF showed bop3 exhibit an $IC_{50}$ of 1.97 $\pm$ 0.17 nM (Fig. 14D). Finally, the inventors sought to repeat both the proliferation and competitive inhibition assays in the healthy donor-derived primary hematopoietic (CD34$^+$) stem cells, as a more realistic readout system. As expected, no discernible proliferative activity was observed following treatment with bop3 alone at concentrations of 0.5 µg/mL or 1 µg/mL (Fig. 14E). In contrast, a pronounced inhibition of HSPCs stimulated with 1 ng/mL rhG-CSF was apparent at the same concentrations of bop3 (Fig. 14F). A moderate inhibitory effect was noticeable even at the lower concentration of 100 ng/mL bop3 (Fig. 15F). An inert protein with a similar structural (moevan_control) was also purified and tested under the same conditions, and did not show any measurable activity at the highest used concentration (i.e. 1 µg/mL; Fig. 14). Furthermore, bop3 was not toxic to any of the used cells, highlighting the specificity of its G-CSFR antagonism.

**Claims**

1. A polypeptide configured to assemble into a binder of granulocyte-colony stimulating factor receptor (G-CSFR), which is a non-immunoglobulin-derived polypeptide.

2. The polypeptide of claim 1, wherein the binder is an inhibitor of G-CSFR.

3. The polypeptide of claim 1 or 2, which comprises an amino acid chain having approx. 60 - approx. 180 amino acids, preferably approx. 80 - approx. 160 amino acids, further preferably approx. 90 - approx. 150 amino acids, further preferably of approx. 100 - approx. 140 amino acids, further preferably of approx. 110 - approx. 130 amino acids, most preferably of approx. 124 amino acids.

4. The polypeptide of any of the preceding claims, which comprises the following amino acid sequence SEQ ID NO: 1:

MAALDAALYEIYDGLILYQQRLKSLEGISPELGPALDALRX$_1$X$_2$MAX$_3$FAX$_4$X$_5$

MAQAMEEGLDSLPQX$_6$FLX$_7$X$_8$ALX$_9$X$_{10}$IRX$_{11}$IQADAAALREKLAATYKGNDR

AAAAQSIARKLEEMLEKAYQILRHLAAA,

wherein

$X_1$ = Y, L, W
$X_2$ = D
$X_3$ = D
$X_4$ = I, R, Y, C
$X_5$ = L
$X_6$ = R, 5
$X_7$ = R, Y, W
$X_8$ = K
$X_9$ = E
$X_{10}$ = M
$X_{11}$ = K, W.

5. The polypeptide of any of the preceding claims, which comprises any of the following amino acid sequences: SEQ ID NO: 2 (bop3), SEQ ID NO: 3 (bop1), SEQ ID NO: 4 (bop2), SEQ ID NO: 5 (boa1), SEQ ID NO: 6 (boa2), SEQ ID NO: 7 (bv21), SEQ ID NO: 8 (bv22), SEQ ID NO: 9 (bv23), SEQ ID NO: 10 (bv24).

6. The polypeptide of any of claims 1-5 for use as a medicament, preferably in the treatment of a disease, further preferably selected from the group consisting of:

cancer, including leukemia and acute myeloid leukemia; cardiovascular disease; inflammatory disease, including rheumatoid arthritis; and neurologic disease.

7. A protein comprising the polypeptide of any of claims 1-6.

8. The protein of claim 7, comprising:

a) one polypeptide chain;
b) a bundle of four α-helices; and
c) three amino acid linkers that connect contiguous bundle-forming α-helices that are located on the polypeptide chain, wherein each amino acid linker has a length between 2 and 15 amino acids;

wherein the protein comprises one or more G-CSFR binding sites, and
wherein the protein has a melting temperature ($T_m$) of at least 90 °C.

9.  The protein of claim 8, wherein $T_m$ is at least 100 °C, preferably at least 110 °C, preferably

the protein binds to G-CSFR with a dissociation constant $K_D$ of less than 100 nM, further preferably less than 50 nM, further preferably less than 10 nM, further preferably less than 5 nM, further preferably less than 4 nM, further preferably less than 3 nM, further preferably less than 2 nM, further preferably less than 1 nM, preferably
the protein has an inhibitory activity of G-CSF-induced proliferation, further preferably at a half maximal inhibitory concentration ($IC_{50}$) of approx. 1 - 40 nM, preferably 1 - 30 nM, further preferably 1 - 20 nM, preferably
the protein has a molecular weight of ≤ 14 kDa.

10. The protein of any of claims 7-9, wherein the polypeptide chain comprises an amino acid sequence having at least 60%, 70%, 80%, 90% or 100% amino acid sequence identity with an amino acid sequence selected from the group consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 and SEQ ID NO: 10.

11. A nucleic acid molecule encoding the polypeptide of any of claims 1-6, or the protein of any of claims 7-10, optionally linked to a promoter sequence.

12. A recombinant host cell comprising the polypeptide of any of claims 1-6, or the protein of any of claims 7-10, and/or the nucleic acid molecule of claim 11.

13. A pharmaceutical composition comprising the polypeptide according to any of claims 1-6, or the protein of any of claims 7-10, the nucleic acid molecule of claim 11, and/or the recombinant host cell of claim 12.

14. A kit comprising:

(a) a container comprising the polypeptide according to any of claims 1-6, or the protein of any of claims 7-10, the nucleic acid molecule of claim 11, the recombinant host cell of claim 12, or the pharmaceutical composition according to claim 13, in solution or in lyophilized formulation;
(b) optionally, a second container containing a diluent or reconstituting solution for the lyophilized formulation;
(c) optionally, instructions for (i) use of the solution or (ii) reconstitution and/or use of the lyophilized formulation.

15. A use of the polypeptide according to any of claims 1-6 for reconstituting a G-CSFR-binding and/or G-CSFR-inhibiting protein, preferably a monomeric protein.

Fig. 1

Fig. 2

Fig. 3

**Fig. 4**

**Fig. 5**

Fig. 6

A)

B)

Fig. 7

Fig. 8

A)

Design model
Crystal structure

B) Primary binding site

C) Secondary binding site

G-CSF
Design
Structure

G-CSF
Design
Structure

Fig. 9

Fig. 10

**A)**

PCR with two oligo pools
coding for desired mutations

☆ = mutation site

Blunt end
ligation → Boskar4 library
with ~ 6×10⁶
transformants

**B)** **Sanger sequencing of final library**

| Mutation # | 1 | 2 | 3 | 4 | 5 | 6 | 7 | 8 | 9 | 10 | 11 |
|---|---|---|---|---|---|---|---|---|---|---|---|
| Amino acid and position in Boskar4 (7NY0) | L38 | D39 | D42 | T45 | T46 | S61 | L64 | K65 | E68 | Q69 | K72 |
| Unmutated codon | CTG | GAT | GAT | ACC | ACG | AGC | CTG | AAA | GAA | CAG | AAA |

**C)** **FACS enrichment after sort (as)**

Fluorescence PE-A

**D)** **Single variant screen**

After sort 3 with 10 nM BioGCSFR

| | 0 1 | 2 | 3 | 4 | 5 | 6 |
|---|---|---|---|---|---|---|
| A | -0.51 | -0.41 | -0.36 | 0.16 | 0.86 | 0.36 |
| B | -0.01 | 0.22 | 0.50 | 0.16 | -0.96 | -1.32 |
| C | -0.55 | -0.26 | -0.02 | 0.82 | 0.65 | 0.44 |
| D | 1.69 | -0.24 | -1.46 | -0.11 | 0.43 | 0.40 |
| E | -1.13 | -0.88 | -0.25 | 3.27 | -0.40 | 0.88 |
| F | 3.41 | 1.51 | -0.72 | 0.49 | 0.86 | -1.41 |
| G | -0.13 | 0.02 | -0.54 | -0.27 | -0.43 | -0.51 |
| H | -0.81 | -0.75 | -0.39 | -1.13 | -0.86 | -0.31 |

After sort 5 with 3 nM BioGCSFR

| | 0 1 | 2 | 3 | 4 | 5 | 6 |
|---|---|---|---|---|---|---|
| A | -0.09 | -0.14 | 0.33 | 0.31 | 0.29 | 0.03 |
| B | -0.26 | -0.45 | 0.14 | 0.53 | 0.44 | -0.02 |
| C | -0.46 | 0.02 | 0.24 | 0.38 | -2.04 | -0.23 |
| D | -0.38 | 0.73 | 0.55 | -0.83 | -0.65 | 0.27 |
| E | -0.78 | -0.12 | 1.29 | -0.08 | -0.04 | 0.13 |
| F | -0.05 | -0.11 | -0.08 | 0.13 | -0.85 | -0.75 |
| G | 0.48 | -0.32 | -0.32 | -0.03 | 2.02 | 1.17 |
| H | 4.73 | -0.91 | -0.72 | -0.72 | -0.55 | -2.25 |

**Fig. 11**

Fig. 12

Fig. 13

**Fig. 14**

Fig. 15

Europäisches
Patentamt

European
Patent Office

Office européen
des brevets

# EUROPEAN SEARCH REPORT

**Application Number**

EP 24 18 5454

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/123033 A1 (MAX PLANCK GESELLSCHAFT [DE]; UNIV EBERHARD KARLS TUEBINGEN [DE]) 24 June 2021 (2021-06-24) * sequence 5 * * examples 3, 9, 10, 12 * * page 131; table 6 * ----- | 1,3,6-15 | INV. A61P7/00 A61K38/00 A61K38/18 C07K14/535 |
| A | ELGAMACY M ET AL: "De novo design of cytokines, antikines, and novokines", 34. JAHRESTAGUNG DER KIND-PHILIPP-STIFTUNG FÜR PÄDIATRISCH ONKOLOGISCHE FORSCHUNG, vol. 234, 1 May 2022 (2022-05-01), XP093234505, DOI: 10.1055/s-0042-1748728 Retrieved from the Internet: URL:https://www.thieme-connect.de/products/ejournals/html/10.1055/s-0042-1748728> * the whole document * ----- | 1-15 | |
| T | ULLRICH TIMO ET AL: "A strategy to design protein-based antagonists against type I cytokine receptors", PLOS BIOLOGY, vol. 22, no. 11, 26 November 2024 (2024-11-26), page e3002883, XP093234508, US ISSN: 1545-7885, DOI: 10.1371/journal.pbio.3002883 * the whole document * ----- -/-- | | TECHNICAL FIELDS SEARCHED (IPC) A61P A61K C07K |

The present search report has been drawn up for all claims

| Place of search | Date of completion of the search | Examiner |
|---|---|---|
| The Hague | 16 December 2024 | Schmitz, Till |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

  .............................................................................

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 24 18 5454

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | ULLRICH TIMO ET AL: "Supplementary material - A strategy to design protein-based antagonists against type I cytokine receptors", PLOS BIOLOGY, vol. 22, no. 11, 26 November 2024 (2024-11-26), page e3002883, XP093234510, US ISSN: 1545-7885, DOI: 10.1371/journal.pbio.3002883 * table B * | | |
| A,D | KAREN SCALZO-INGUANTI ET AL: "A neutralizing anti-G-CSFR antibody blocks G-CSF-induced neutrophilia without inducing neutropenia in nonhuman primates", JOURNAL OF LEUKOCYTE BIOLOGY, vol. 102, no. 2, 17 May 2017 (2017-05-17), pages 537-549, XP055638085, US ISSN: 0741-5400, DOI: 10.1189/jlb.5A1116-489R * the whole document * | 1-15 | |
| A,D | WO 2012/171057 A1 (CSL LTD [AU]; NASH ANDREW DONALD [AU] ET AL.) 20 December 2012 (2012-12-20) * the whole document * | 1-15 | TECHNICAL FIELDS SEARCHED (IPC) |

The present search report has been drawn up for all claims

| Place of search | Date of completion of the search | Examiner |
|---|---|---|
| The Hague | 16 December 2024 | Schmitz, Till |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                       

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 5454

This annex lists the patent family members relating to the patent documents cited in the above-mentioned European search report.
The members are as contained in the European Patent Office EDP file on
The European Patent Office is in no way liable for these particulars which are merely given for the purpose of information.

16-12-2024

| Patent document cited in search report | Publication date | Patent family member(s) | Publication date |
|---|---|---|---|
| WO 2021123033 A1 | 24-06-2021 | AU 2020406137 A1 | 16-06-2022 |
| | | CA 3159912 A1 | 24-06-2021 |
| | | EP 4076651 A1 | 26-10-2022 |
| | | US 2023227520 A1 | 20-07-2023 |
| | | WO 2021123033 A1 | 24-06-2021 |
| WO 2012171057 A1 | 20-12-2012 | AU 2012269720 A1 | 21-03-2013 |
| | | BR 112013031943 A2 | 22-11-2016 |
| | | CA 2838246 A1 | 20-12-2012 |
| | | CN 103649123 A | 19-03-2014 |
| | | DK 2718326 T3 | 26-10-2020 |
| | | EP 2718326 A1 | 16-04-2014 |
| | | ES 2828482 T3 | 26-05-2021 |
| | | JP 6187777 B2 | 30-08-2017 |
| | | JP 2014519517 A | 14-08-2014 |
| | | KR 20140041717 A | 04-04-2014 |
| | | MX 343580 B | 10-11-2016 |
| | | NZ 617725 A | 27-02-2015 |
| | | RU 2014100642 A | 20-07-2015 |
| | | SG 195043 A1 | 30-12-2013 |
| | | US 2012321630 A1 | 20-12-2012 |
| | | US 2016031998 A1 | 04-02-2016 |
| | | US 2017226214 A1 | 10-08-2017 |
| | | US 2019055313 A1 | 21-02-2019 |
| | | US 2021009701 A1 | 14-01-2021 |
| | | WO 2012171057 A1 | 20-12-2012 |

EPO FORM P0459

For more details about this annex : see Official Journal of the European Patent Office, No. 12/82

## REFERENCES CITED IN THE DESCRIPTION

**Patent documents cited in the description**

- WO 2012171057 A1

**Non-patent literature cited in the description**

- **SCALZO-INGUANTI et al.** 164: CSL324, a humanised anti G-CSFR antibody, can inhibit neutrophil migration while not impaction on neutrophil number or effector functions.. *Cytokine*, 2014, vol. 70 (1), 68
- **SCALZOLNGUANTI et al.** A neutralizing anti-G-CSFR antibody blocks G-CSF-induced neutrophilia without inducing neutropenia in nonhuman primates. *Journal of leukocyte biology*, 2017, vol. 102 (2), 537-549
- **MCRAE et al.** Blockade of the G-CSF Receptor Is Protective in a Mouse Model of Renal Ischemia-Reperfusion Injury.. *The Journal of Immunology*, 2020, vol. 205 (5), 1433-1440
- **GAMELL et al.** CSL324, a granulocyte colony-stimulating factor receptor antagonist, blocks neutrophil migration markers that are upregulated in hidradenitis suppurativa.. *British Journal of Dermatology*, 2023, vol. 188 (5), 636-648
- **MCGUFFIN et al.** The PSIPRED protein structure prediction server. *Bioinformatics*, 2000, vol. 16 (4), 404-405
- **YANG et al.** SPIDER2: A package to predict secondary structure, accessible surface area, and main-chain torsional angles by deep neural networks. *PSSPred*, 2016, https://zhanglab.ccmb.med.umich.edu/PSSpred
- **WANG et al.** Protein secondary structure prediction using deep convolutional neural fields. *Scientific Reports*, 2016, vol. 6, 18962
- **NEEDLEMAN ; WUNSCH**. *J. Mol. Biol.*, 1970, vol. 48, 443-453
- **RICE ; P. LONGDEN, I ; BLEASBY, A.** EMBOSS: The European Molecular Biology Open Software Suite. *Trends in Genetics*, 2000, vol. 16 (6), 276-277